# EUROPEAN PATENT SPECIFICATION

(11) **EP 0 505 680 B1**
(45) Date of publication and mention of the grant of the patent: **26.04.1995**
(21) Application number: 92101196.1
(22) Date of filing: 27.01.1992
(51) Int. Cl.: C07K 7/02, C07K 14/655, A61K 38/31

(54) **Octapeptide or heptapeptide derivatives, a process for preparing them as well as medicaments containing these compounds and the use of them**
Octapeptid oder Heptapeptidderivate, Verfahren zu deren Herstellung sowie Medikamente, die diese Verbindungen enthalten und Verwendung derselben
Octapeptidyl et heptepeptidyl dérivés, procédé pour les préparer aussi comme médicaments qui les contiennent et leur utilisation

(30) Priority: 25.01.1991 HU 27291
(43) Date of publication of application: 30.09.1992
(73) Proprietor: BIOSIGNAL Kutato-Fejleszto Kft., H-1022 Budapest (HU)
(72) Inventor: Kéri, György, Dr., H-1021 Budapest (HU); Mezö, Imre, Dr., H-1156 Budapest (HU); Horváth, Aniko, Dr., H-1038 Budapest (HU); Vadász, Zsolt, Dr., H-1141 Budapest (HU); Teplán, István, Dr., H-1026 Budapest (HU); Balogh, Agnes, H-1141 Budapest (HU); Csuka, Orsolya, Dr., H-1127 Budapest (HU); Bökönyi, Gyöngyi, H-1031 Budapest (HU); Szöke, Balázs, Dr., H-1088 Budapest (HU); Horváth, Judit, Dr., H-7624 Pécs (HU); Idei, Miklos, Dr., H-1133 Budapest (HU); Seprödi, János, Dr., H-1028 Budapest (HU)
(74) Representative: Beszédes, Stephan G., Dr.

(56) References cited:
- EP-A- 0 203 031
- EP-A- 0 215 171
- EP-A- 0 277 419
- EP-A- 0 298 732
- EP-A- 0 389 180
- Proc. Natl. Acad. Sci. USA, 86(6), pages 2003-2007 (1986)
- Greenstein & Winitz, Chemistry of amino acids, Wiley, London, 1961, vol. 1, pages 5-6
- Current opinion in therapeutic patents, 2(12), Dec. 1992, pages 1993-1994
- Biochemical and biophysical research communications, 191(2), 1993, pages 681-687

## Description

The invention is concerned with novel octapeptide or heptapeptide amide derivatives, a process for preparing them as well as medicaments containing these compounds and the use of them.

Somatostatin, a cyclic tetradecapeptide (SRIF), being an inhibitor of secretion of the growth hormone (GH) was originally isolated from the hypothalamus [Brazeau et al.: Science 179, 77 (1973)]. Somatostatin has a very broad spectrum of biological effects, participates in a high number of biological processes and in the majority of cases it plays a role of an inhibitory factor (it inhibits e.g. the release of prolactin, insulin, glucagon, gastrin, secretin and cholecystoquinine) [S. Reichlin: Somatostatin, N. Eng. J. Med. 309, 1495 and 1536 (1983)].

One of the most important effects of somatostatin being a growth-inhibiting factor consists in its capability to influence various forms of pathological cell growth. It is well known from the literature that it exerts an inhibitory action on the growth of cancerous cells [A. V. Schally: Cancer Rs. 48, 6977 (1988); Taylor et al.: Biochem. Biophys. Res. Commun. 153, 81 (1988)]. Likely, somatostatin exerts its antagonizing action on growth factors related to cancerous processes. It has been shown by recent investigations that somatostatin and some somatostatin analogues are capable to activate the tyrosine phosphatase enzyme which antagonizes the effect of tyrosine kinases playing a very important role in the tumorous transformation [A. V. Schally: Cancer Res. 48, 6977 (1988)]. The importance of tyrosine kinases is supported by the fact that the majority of oncogens codes for tyrosine kinase and the major part of the growth factor receptors is tyrosine kinase [Yarden et al.: Ann. Rev. Biochem. 57, 443 (1989)].

Native somatostatin has a very short duration of effect in vivo since it is rapidly inactivated by endo- and exopeptidases. A high number of novel analogues have been prepared in order to enhance the duration of effect, biological activity and selectivity of this hormone. Most of the active analogues contain a cycle and a peptide chain which is shorter than the original one. The first cyclic hexapeptide showing the whole effects of somatostatin was synthetized by Veber et al. [Nature 292, 55 (1981)]. As a continuation newer and more effective cyclic hexa- and octapeptides have been synthetized which possess the whole spectrum of effects of somatostatin [Veber et al.: Life Sci. 34, 1371 (1984); Murphy et al.: Biochem. Biophys. Res. Commun. 132, 922 (1985); Cai et al.: Proc. Natl. Acad. Sci. USA 83, 1896 (1986)].

Moreover from EP-A 203 031 there have been known biologically active lysine containing octapeptides of the formula 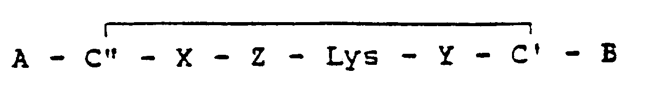
wherein
- A: represents an L, D or DL amino-acid selected from the group consisting of Ala, Val, Phe, p-Cl-Phe, Trp, Pro, Ser, Thr, Glu, Gly, Beta Ala, Abu, N-Me Ala, 5-F-Trp, 5-Br-Trp, 5-Cl-Trp, their acetylated derivatives or a pharmaceutically acceptable acid addition salt thereof;
- B: represents an L, D or DL amino acid amide selected from the group consisting of Thr NH₂, Val NH₂, Pro NH₂, HO-Pro NH₂, Ser NH₂, Tyr NH₂, Trp NH₂, 5-F-Trp NH₂, For-Trp NH₂, Ala NH₂, Gly NH₂, Me Ala NH₂;
- X: represents L-Phe or L-Tyr,
- Y: represents L-Thr or L-Val;
- Z: is L, D or DL-5-F-Trp, 5-Br-Trp, 5-Cl-Trp, 5-1-Trp or D-Trp;
and
- C'' and C': represent L or D Cys, Abu, Asp or Lys; provided that where C' is Cys, C'' is also Cys and where C' or C'' are other than Cys, C'' is different from C' and is other than Cys and the pharmaceutically acceptable acid addition salts thereof; are useful as agents for inhibiting the release of growth hormone, for the treatment of gastrointestinal disorders and for therapy of certain cancers and the management of diabetes.

The problem underlying to the invention is to create novel somatostatin analogues showing a more advantageous and/or more selective pharmacological action, particularly in treating mammals, including man, suffering from tumour disease, in comparison to that of the known compounds, a process for preparing them as well as medicaments containing these compounds and the use of them.

Surprisingly this has been solved by the invention.

The invention is based on the recognition that the effect of octapeptide and heptapeptide analogues of somatostatin can be strengthened when the amino acid in 1-position is replaced by a substituent of strongly hydrophobic character and having a structure inhibiting the activity of exopeptidases. This substitution can preferably be combined with the replacement of the amino acid in 3-position by various aromatic or heterocyclic amino acids; or with the replacement of the amino acid in 4-position by an aromatic D-amino acid, aromatic aminocarboxylic acid, aminosuccinimide or a β-aspartyl group, optionally substituted on its α-carboxyl group; or with the replacement of the amino acid in 6-position by an amino acid, bearing an alkyl or aralkyl side chain, or substituted derivatives thereof or proline or β-alanine; or with omission of the amino acid in 6-position; or with replacement of the amino acid in 8-position by an aromatic amino acid or a ring-substituted derivative thereof or by threonine.

A further basis of the invention is the recognition that, when an anthranilyl substitution, for example, in compound of formula 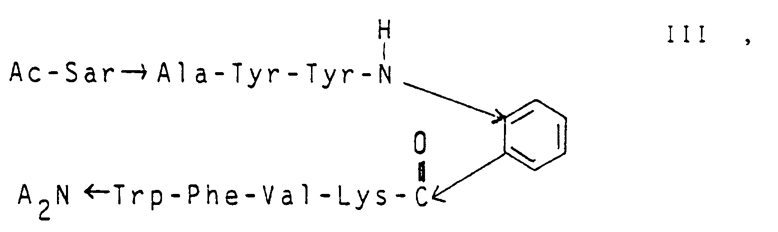
m-aminobenzoyl substitution, for example, in compound of formula 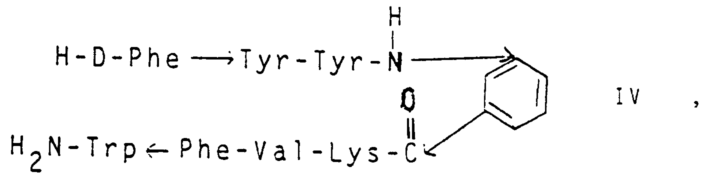
or aminosuccinyl substitution, for example, in compound of formula 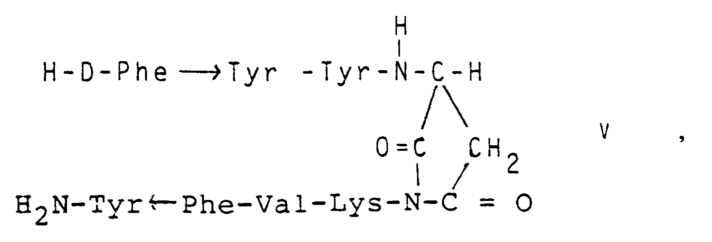
is used, the compound has not to be cyclized since these replacements inhibit in some cases the free steric rotation of a given peptide (US patent 4,758,552; HU patent 194,280); otherwise the rotation is prevented by the cyclized form, too, for example, in compound of formula 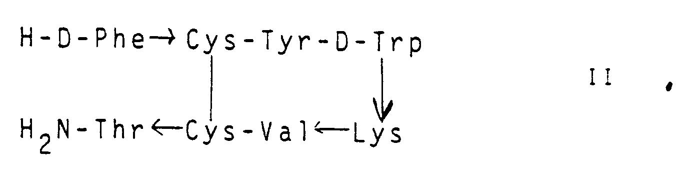
Thus a subject matter of the invention are octapeptide or heptapeptide amide derivatives of the general formula 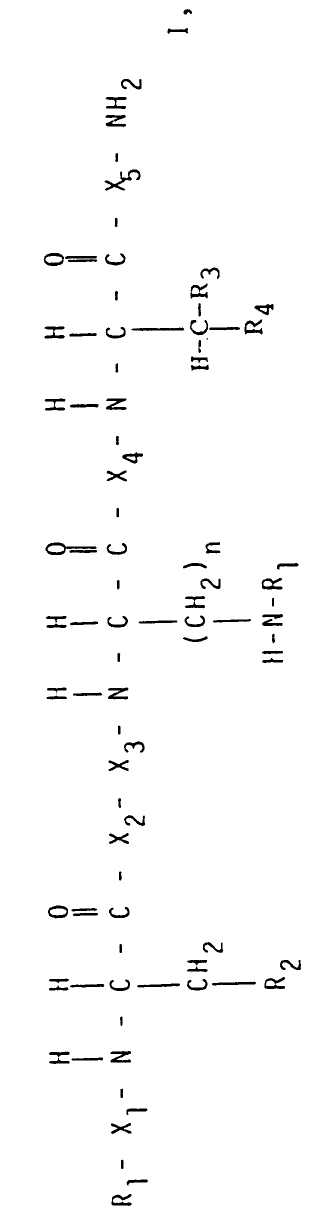
wherein
- X₁: stands for an aromatic D-amino acid rest or a derivative thereof ring-substituted by halogen and/or hydroxyl; or a D-phenylglycyl, p-hydroxyphenylglycyl, o-aminobenzoyl, m-aminobenzoyl, D-tetrahydroisoquinolylcarbonyl or sarcosylalanyl; or a 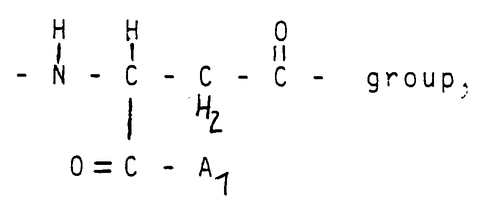 in which latter
- A₁: means the rest of an aromatic, heterocyclic or aliphatic amine having a primary or secondary amino group or means a primary or secondary aromatic, heterocyclic or aliphatic amino group or means an alkoxy, aryloxy or aralkyloxy group;
- X₂: represents an aromatic amino acid rest or a derivative thereof ring-substituted by halogen and/or hydroxyl; or a histidyl group;
- X₃: means a D-tryptophyl, o-aminobenzoyl, m-aminobenzoyl, aspartyl, D-aspartyl, β-aspartyl, β-D-aspartyl or aminosuccinyl group; or a 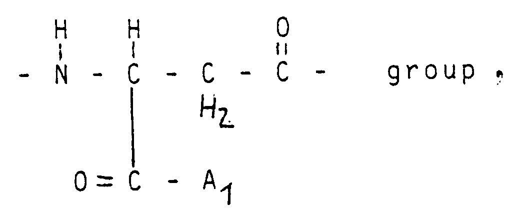 in which latter
- A₁: is as defined above;
- X₄: stands for an amino acid rest bearing an alkyl or aralkyl side chain, or a derivative thereof substituted by a hydroxyl or methyl group in β-position; or a prolyl or β-alanyl group; or a valence bond;
- X₅: means a heterocyclic amino acid rest or a derivative thereof ring-substituted by halogen and/or hydroxyl; or a threonyl group;
- R₁: represents hydrogen or a 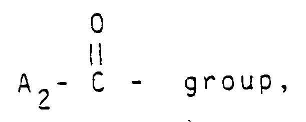 in which latter
- A₂: means hydrogen or a C₁₋₄ alkyl group, optionally substituted by halogen;
- R₂: means hydrogen, -S- or a -S-acetamidomethyl, phenyl or p-hydroxyphenyl group;
- R₃: stands for hydrogen or hydroxyl;
- R₄: means a phenyl or p-hydroxyphenyl group; or -S- or a -S-acetamidomethyl group when the meaning of R₂ is the same group; or a methyl group when R₃ is hydroxyl, in case of R₂ and R₄ each being -S- one bond each of them are united to a sole bond representing a bridge between R₂ and R₄
and
- n: is 1, 2, 3 or 4,
with the provisos that
a) if
   - X₁: stands for D-β-Nal or D-Phe, optionally substituted by halogen or a hydroxy group,
   - X₂: means Tyr or Phe
   - X₃: represents D-Trp,
   - X₅: stands for Trp or Thr and
   - R₂ and R₄: each are -S-, one bond each of them being united to a sole bond representing a bridge between R₂ and R₄
   - X₄: is different from Val, Thr or Ser,
b) if
   - X₄: stands for Val or Thr or Ser and
   - X₁: means an aromatic D-amino acid residue or a derivative thereof ring-substituted by halogen or hydroxy
   - X₂: represents an aromatic amino acid rest or a derivative ring-substituted by halogen or hydroxy
at least one of the following conditions is fulfilled
α)
   - X₃: is different from D-Trp
   or
β)
   - R₂ and R₄: together have a meaning different from a - S - S - group (disulfide bridge),
and acid addition salts thereof.

The abbreviations used in the formulae are in agreement with the nomenclature accepted in the peptide chemistry, which has been published e.g. in J. Biol. Chem. 241, 527 (1966). According to this the abbreviations occurring in the description are as follows:

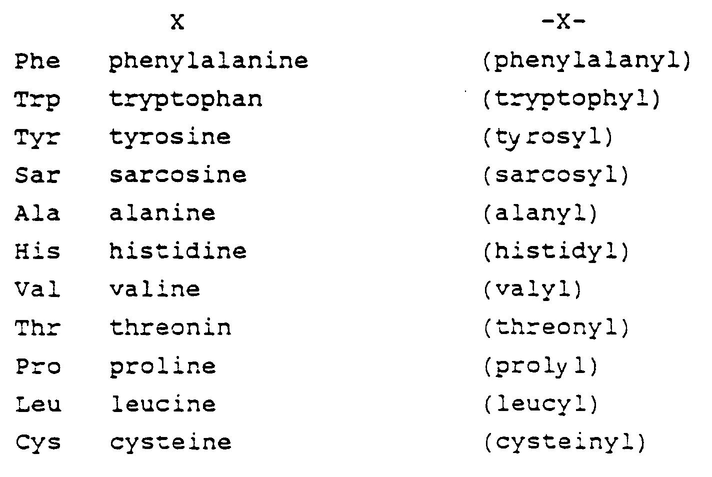

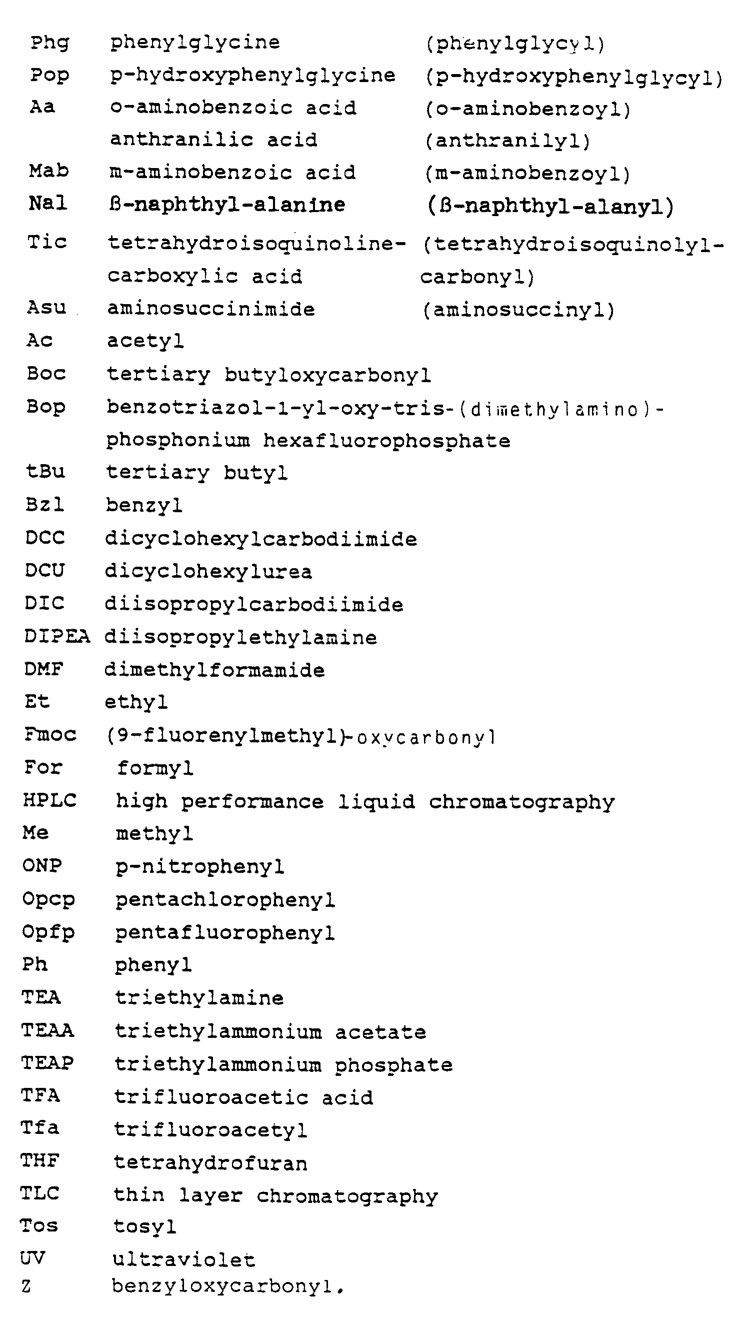

Preferably the D-amino acid rest which may be represented by X₁ is a D-phenylalanyl, D-naphthylalanyl, preferably D-2-naphthyl-alanyl, or D-tryptophyl rest, above all the first one, and/or the amino acid rest which may be represented by X₂ is a tyrosyl rest and/or the halogen by which the rest of the D-amino acid derivative which may be represented by X₁ and/or the rest of the amino acid derivative which may be represented by X₂ may be ring-substituted is iodine, particularly preferably the rest of the D-amino acid derivative which may be represented by X₁ being a D-diiodtyrosyl rest and/or the rest of the amino acid derivative which may be represented by X₂ being a diiodtyrosyl rest.

Furthermore it is preferred that the rest of the amine which may be represented by A₁ is an indolinyl or phenylamino rest, the alkoxy group which may be represented by A₁ is such having from 1 to 4, particularly 1 or 2, carbon atom(s), the aryloxy group which may be represented by A₁ is a phenyloxy group or the aralkyloxy group which may be represented by A₁ is such having from 1 to 4, particularly 1 or 2, carbon atom(s) in the alkyl part and having as aryl part a phenyl part.

Moreover it is preferred that the alkyl side chain of the amino acid rest or its derivative which may be represented by X₄ is such having from 1 to 4, particularly 3 or 4, carbon atom(s) or the aralkyl side chain or the amino acid rest or its derivative which may be represented by X₄ is such having from 1 to 4, particularly 1 or 2, carbon atom(s) in the alkyl part and having as aryl part a phenyl part, particularly the amino acid rest which may be represented by X₄ being a leucyl, valyl, isoleucyl, norvalyl, norleucyl, alanyl or seryl rest, above all one of the first two, and/or the aromatic amino acid rest which may be represented by X₅ is a tryptophyl or phenylalanyl rest, above all the first one, or the halogen by which the rest of the derivative of the aromatic amino acid which may be represented by X₅ may be substituted is iodine, particularly preferably the rest of the derivative of the aromatic amino acid which may be represented by X₅ being a tyrosyl or diiodtyrosyl rest, and/or the C₁₋₄ alkyl group which may be represented by A₂ is such having 1 or 2 carbon atoms or the halogen by which the C₁₋₄ alkyl group which may be represented by A₂ may be substituted is fluorine the number of it being preferably 3, and/or n is 3 or 4, above all the latter one.

Particularly preferred compounds are 
β-aspartyl-(indolinyl)-Cys-
-Tyr-D-Trp-Lys-Val-Cys-Thr-NH2,
D-Phe-Cys-Tyr-D-Trp-Lys-
-Cys-Thr-NH2, D-Phe-Cys-Tyr-D-Trp-Lys-Leu-Cys-Thr-
-NH2, D-Phe-Cys-Tyr-β-Asp(indolinyl)-Lys-Leu-Cys-
-Thr-NH2,
D-Phe-Tyr-Tyr-Aa-Lys-Val-Phe-Trp-NH₂, Ac-Sar-Ala-Tyr-Tyr-Aa-Lys-Val-Phe-Trp-NH₂, Pop-Tyr-Tyr-Aa-Lys-Val-Phe-Trp-NH₂ and Aa-Cys(Acm)-Tyr-D-Trp-Lys-Val-Cys(Acm)-Trp-NH₂.

Suitably the acid addition salts of the octapeptide or heptapeptide derivatives according to the invention are such with pharmaceutically acceptable acids.

A subject matter of the invention is also a process for the preparation of the compounds according to the invention which is characterized in that using the method of the solid-phase peptide synthesis, the protected amino acids are stepwise condensed onto the solid resin carrier through carbodiimide or active ester coupling in the corresponding succession, then the final product is removed from the solid carrier by acidic or alkaline cleavage and the protective groups are removed from the amino acids simultaneously with or before or after cleavage from the resin and, if desired, the octapeptide or heptapeptide amide of the general formula I thus obtained is converted to an acid addition salt by reacting it with an acid and/or, if desired, the free base is liberated from the acid addition salt obtained by reacting it with an alkali and, if desired, the latter is converted to another acid addition salt by reacting it with an acid.

It is suitable to use a benzhydrylamine resin or a chloromethylated polystyrene resin as solid carrier in the process of the invention.

The final product containing protective groups can preferably be splitted off from the resin by using hydrogen fluoride and/or ammonolysis.

As already mentioned, the compounds of the invention have superior pharmacological activity, particularly in treating mammals, including man, suffering from tumour disease, and inhibiting tyrosine kinase activity.

Hence by the invention also there are provided for medicaments which are characterized in that they contain as active ingredient(s) 1 or more compound(s) according to the invention, suitably in admixture with 1 or more carrier(s) and/or additive(s) commonly used in the pharmaceutical technique.

The medicaments according to the invention can be in the form of pharmaceutical preparations. These preparations can be prepared by using methods known per se, and thus suitably by mixing 1 or more compound(s) according to the invention with 1 or more carrier(s) and/or additive(s) commonly used in the pharmaceutical technique and transforming the mixture thus obtained to a pharmaceutical preparation.

The compounds according to the invention were found to be active in the dosage range of 0.01 to 500 »g/kg of body-weight (hereinafter:»g/kg) on mice or 0.5 to 2000 mg/kg on man, respectively.

Hence a further subject matter of the invention is the use of the compounds according to the invention for preparing medicaments, particularly for treating mammals, including man, suffering from tumour disease and/or for inhibiting their tyrosine kinase activity.

The pharmacological effects of the compounds according to the invention are supported by the tests described hereinafter.

### A) Assay of the growth hormone (GH)

The release or inhibition, respectively, of the release of GH were measured on rat hypophysis by using the superfusion method [Vigh et al.: Peptides 5, 241 (1984)]. As control, the GH amount was considered which was released by the GH releasing hormone (GHRH) given in the same dose as the sample.

The activity of the compound according to the invention was expressed as the percentage of decrease or increase, respectively, in the GH amount released by GHRH (Table I).

### B) Assay of the inhibition of cell division

The incorporation of [³H]-thymidine to tumour cells of various origin and measurement of the cell count were carried out according to the method of Kéri et al. [Tumor Biology 9, 315 (1988)]. The biological activity of the compounds according to the invention was expressed as the percentage of inhibition of the labelled thymidine incorporation or increase in the count of untreated cells used as control (Table II).

### C) Measurement of the tyrosine kinase activity

The tyrosine kinase activity was also determined according to the method of Kéri et al. [Tumor Biology 9, 315 (1988)]. The activity of the compounds according to the invention was characterized on the basis of their inhibitory effect in comparison to the incorporation of ³²P isotope to untreated cells used as control (Table III).

### D) Study of effectivity of the compounds on the tumour growth in the metastasis model

The anti-metastatic effect of the compounds was studied on Lewis lung tumour (LLT) cells in muscle-lung and spleen-liver metastasis model as well as on their immunoresistant cell variant (LLT-HH). These experiments were carried out on inbred C₅₇B1 mice of both sexes. The LLT was transplanted into the muscle for developing the muscle-lung metastasis model, whereas a suspension of the tumour cells was injected to the spleen to form the spleen-liver metastasis. The compounds to be tested were administered intraperitoneally (i.p.), intravenously (i.v.), orally (p.o.) or subcutaneously (s.c.) in 0.1 to 10 mg/kg doses daily 1 to 3 times in the 5th to 13th days. The therapeutic effect was evaluated on the basis of the number of metastases in such a way that a sample was taken in the spleen-liver model between the 10th and 14th days, in the muscle-lung model between the 17th and 18th days and the macroscopic metastases in the liver and lungs, respectively, were counted under a stereomicroscope. The efficiency of the compounds was expressed in percentage of the count of metastases determined in the control animals (Table IV).

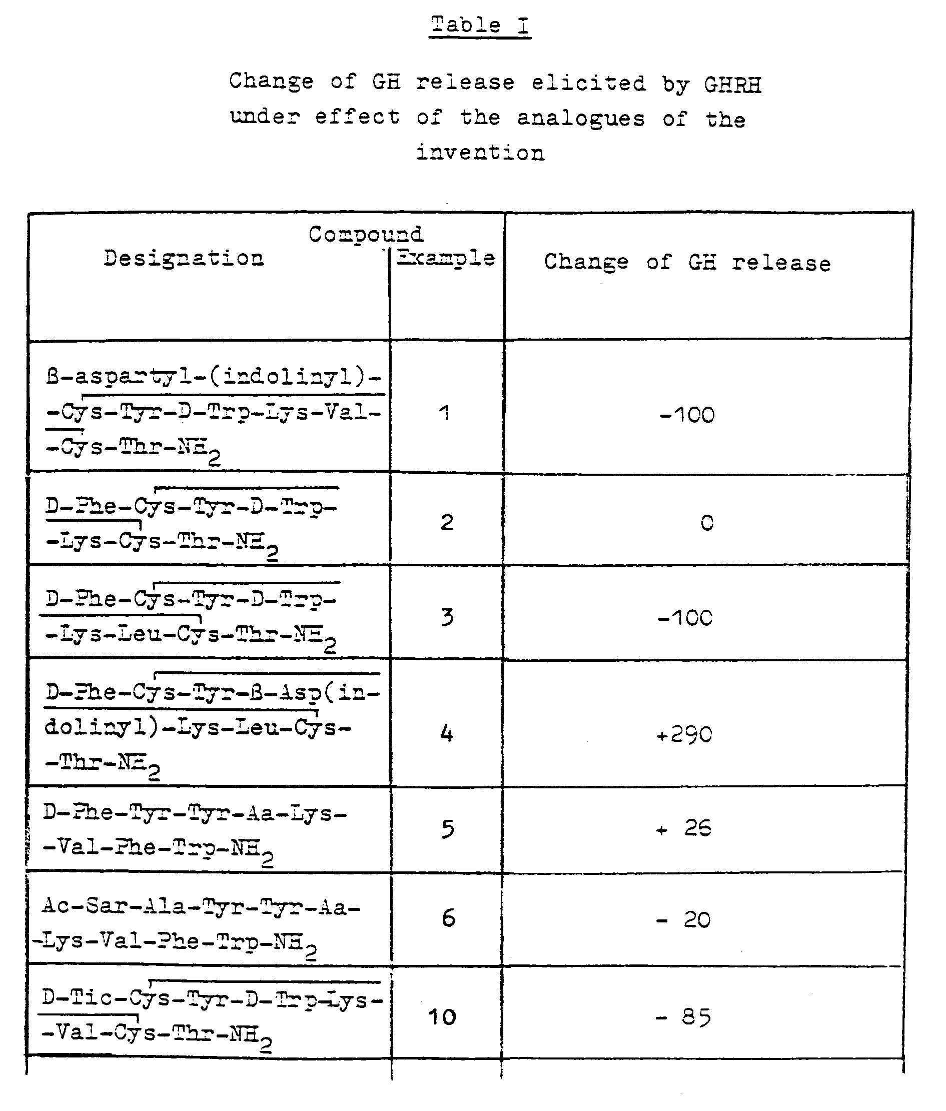

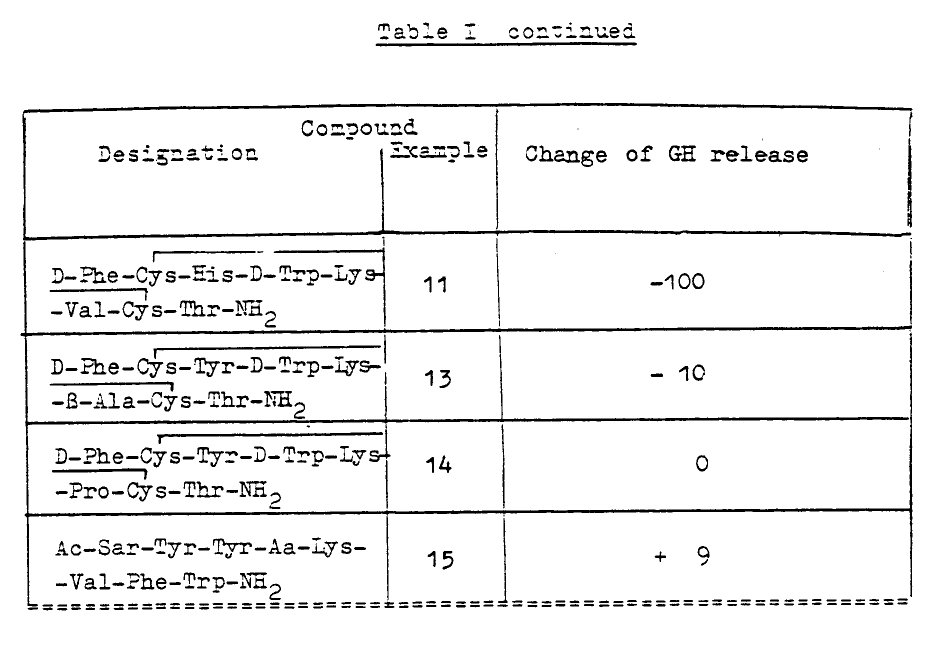

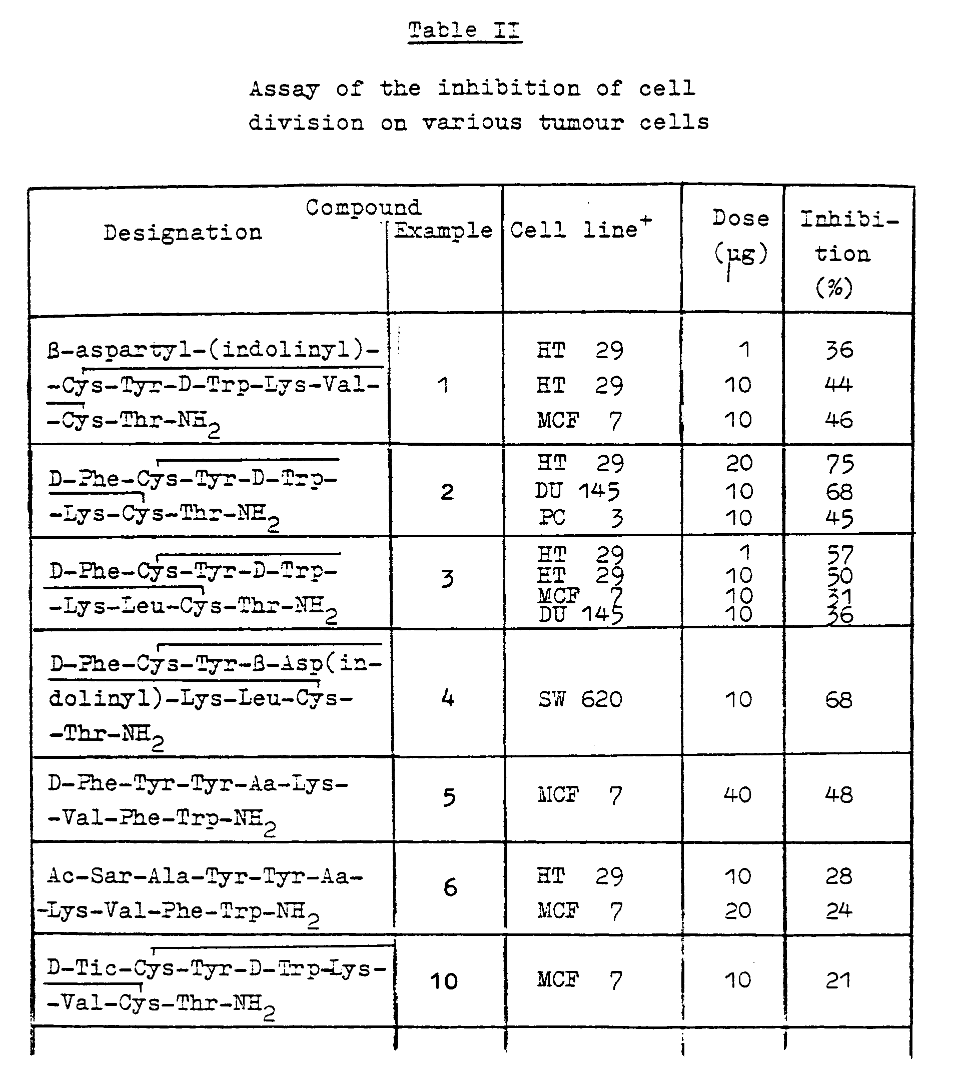

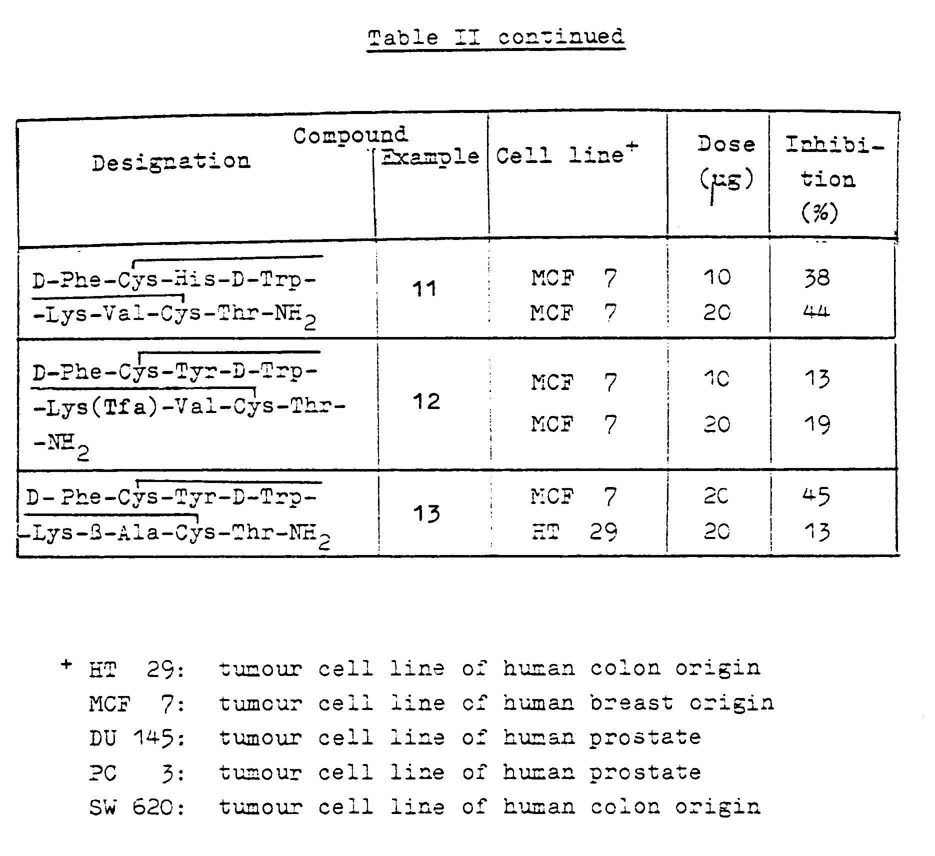

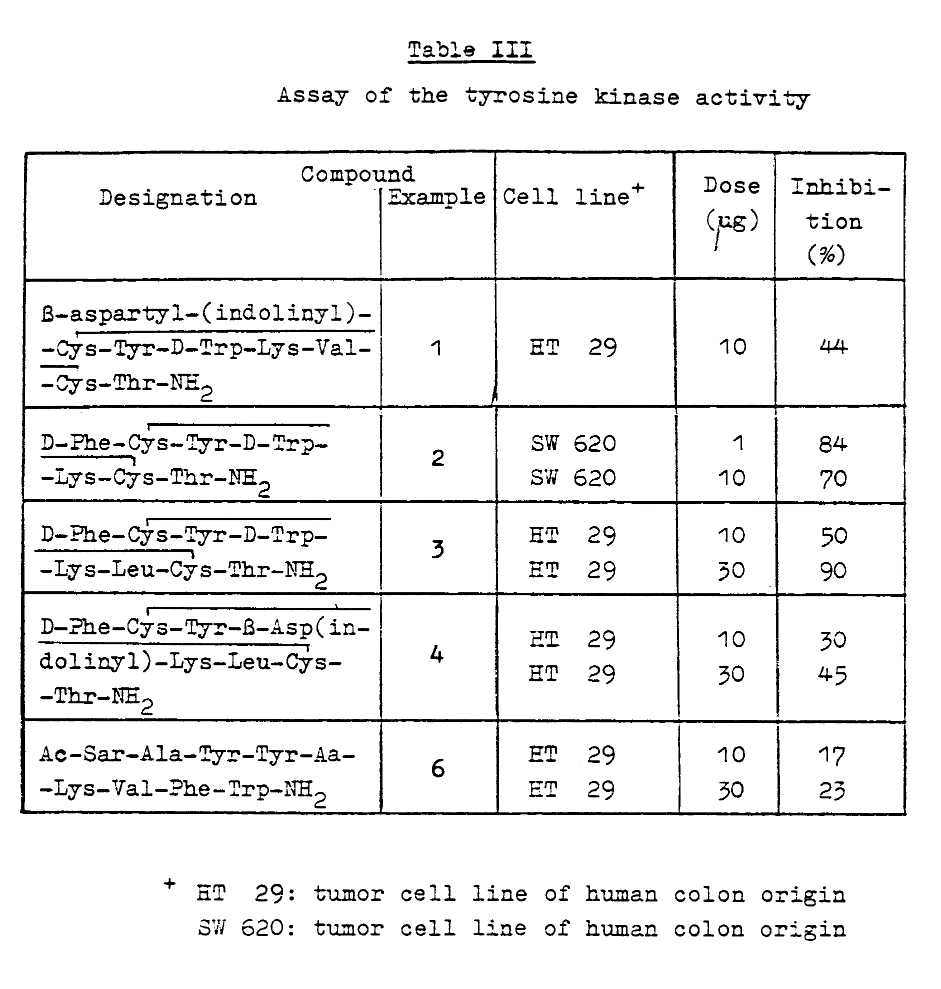

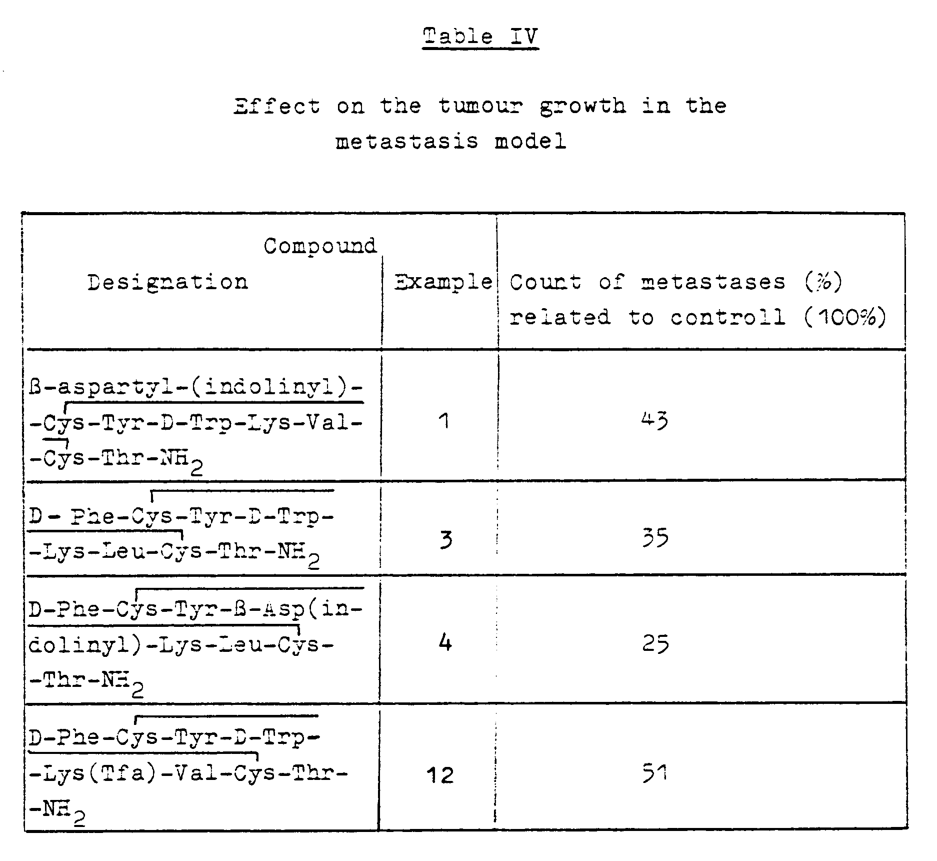

The main advantages of the invention are as follows:
a) The compounds according to the invention can preferably be utilized to inhibit tumour growth or the activity of tyrosine kinase enzymes playing an important role in the tumorous transformation.
b) The compounds according to the invention with the new amino acid combinations are useful also for regulating the release of growth hormone (GH), insulin, glucagon and prolactin and/or for inhibiting tumour growth.
c) From the compounds according to the invention bearing an aminosuccinyl, o- and m-aminobenzoyl substitution, those containing no disulfide bridge do not exert any inhibitory effect on the GH release but inhibit the growth of tumour cells.
d) The compounds according to the invention can be used to inhibit pathological processes, such as psoriasis, elicited by the pathological proliferation of skin cells.
e) The o- and m-aminobenzoyl substituents are devoid of any centre of asymmetry, therefore no racemization can occur during their synthesis. Thus, the preparation of the final product becomes easier and cheaper.
f) The preparation costs of o- and m-aminobenzoic acid are much lower than those of the D-amino acids playing a similar role in somatostatin analogues known up to now.

The invention is illustrated in detail by the following Examples.

In the Examples, the compounds are prepared by methods commonly used in the solid-phase peptide synthesis (Stewart et al.: Solid Phase Peptide Synthesis, 2nd Edition, Pierce Chemical Company, Rockford, Illinois, 1984). The individual amino acids are stepwise bound in the form of their Boc or Fmoc derivatives to a benzhydrylamine or chloromethylated polystyrene resin by the symmetric anhydride or active ester coupling or by the aid of DCC, DIC or BOP reagents.

The progress of the reaction is evaluated by the ninhydrin test [E. Kaiser et al.: Anal. Biochem. 34, 595 (1970)]. The acylation is repeated when a free amino group is detected. The time demand of coupling depends on the amino acids and varies between 1 and 16 hours.

Both the removal of protective groups and cleavage of the peptide from the resin are preferably carried out in a single step by using anhydrous liquid hydrogen fluoride [S. Sakakibara et al.: Bull. Chem. Soc. Japan 40, 2164 (1967)].

If desired, the crude product obtained by HF cleavage is cyclized to form the disulphide bridge.

The crude product is purified by using Sephadex chromatography and/or preparative HPLC method. The purity of the final product is examined by TLC, analytical HPLC and amino acid analysis. The TLC R_{f} values are determined on Kieselgel sheets (DC Alufolien, Merck) by using the solvent mixtures listed hereinafter the ratios being by volume:

| | |
|---|---|
| 1. Ethyl acetate/pyridine/acetic acid/water | 30:20:6:11 |
| 2. Ethyl acetate/pyridine/acetic acid/water | 60:20:6:11 |
| 3. Butanol/pyridine/acetic acid/water | 60:20:6:11 |
| 4. n-Butanol/acetic acid/water | 4:1:2 |
| 5. n-Butanol/acetic acid/water/ethyl acetate | 1:1:1:1 |
| 6. n-Butanol/acetic acid/water | 4:1:1 |
| 7. Isopropanol/1 molar acetic acid | 2:1 |
| 8. Ethyl acetate/pyridine/acetic acid/water | 5:5:1:3 |

In all Examples the percentages of solvents and eluents are by volume.

### Example 1

Preparation of β-aspartyl-(indolinyl)-Cys-Tyr-D-Trp-Lys-
-Val-Cys-Thr-NH2 (Mw: 1076)

0.47 g (0.25 mmol) of benzhydrylamine hydrochloride resin (0.54 millliequivalent/g) is swollen in methylene chloride (CH₂Cl₂) for 90 minutes, then the following cycle is repeated after each acylation carried out with the amino acids.The percentages are by volume.

| Step | Reagents, procedure | Time of stirring (min) |
|---|---|---|
| 1 | CH₂Cl₂; 3 washings | 1 |
| 2 | Mixture containing 33 % of TFA, 5 % of anisole and 62 % of CH₂Cl₂; cleavage | 1 |
| 3 | Mixture containing 33 % of TFA, 5 % of anisole and 62 % of CH₂Cl₂; cleavage | 1 |
| 4 | CH₂Cl₂; 3 washings | 1 |
| 5 | Ethanol; 2 washings | 1 |
| 6 | CH₂Cl₂; 3 washings | 1 |
| 7 | Mixture containing 10 % of TEA and 90 % of CH₂Cl₂; 2 washings | 2 |
| 8 | CH₂Cl₂; 3 washings | 2 |
| 9 | Ethanol; 2 washings | 1 |
| 10 | CH₂Cl₂; 3 washings | 1 |
| 11 | 3 equivalents of Boc-amino acid dissolved in the mixture of CH₂Cl₂ and DMF as well as 3 equivalents of DCC or DIC dissolved in CH₂Cl₂; coupling | 60 minutes to 16 hours |
| 12 | CH₂Cl₂; 3 washings | 1 |
| 13 | Ethanol; 2 washings | 1 |

A ninhydrin test is made after each step. When the result is positive, the cycle is repeated from the 6th step.

0.25 mmol of β-Asp(indolinyl)-Cys(4-Me-Bzl)-Tyr(2-Br-Z)-D-Trp-Lys(Z)-Val-Cys(4-Me-Bzl)-Thr(Bzl) peptide resin is suspended in 3 ml of anisole containing 10 mixed % of p-cresol, and 30 ml of HF gas are condensed onto the mixture. After stirring at 0 °C for 45 minutes HF is removed, the residue is suspended in 200 ml of ethyl acetate, stirred for 15 minutes, then poured into 250 ml of ethyl acetate. After filtration the precipitate is filtered, washed with ethyl acetate, then the peptide is dissolved from the precipitate by filtration, using 500ml of 95 % by volume gas-free acetic acid. Then 9.0 ml of 0.03 M iodine/methanol solution are dropped to the acetic acid solution until it becomes orange yellow. Thereafter, the solution is stirred for one hour. Zinc powder is cautiously added to the solution until the yellow colour disappears, then the zinc powder is filtered and the solution is evaporated. The solid residue is dissolved in 6 ml of 50 % by volume acetic acid and purified on a Sephadex® G-25 column. The elution is followed by using UV absorption measured at 280 nm and TLC. The fractions containing the aimed product are collected, evaporated, then further purified by gradient elution in a preparative HPLC system (column: Whatman Partisil 10 ODS-3 22x250 mm; eluent A: 0.25 N TEAP, pH 2.24; eluent B: 80 % of methanol with 20 % of A). The pure product is desalinized by gradient elution, using 0.02 M NH₄OAc, pH 5 (eluent A) as well as 70 % of methanol with 30 % of eluent A (eluent B) to obtain 120 mg (43 %) of final product. The physical characteristics of this compound are summarized in Table V, the biological effects already have been shown in Tables I, II, III and IV.

### Example 2

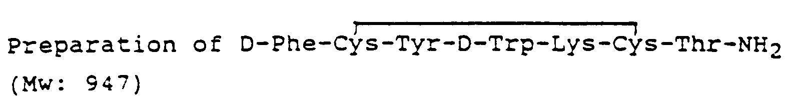
Preparation of D-Phe-Cys-Tyr-D-Trp-Lys-Cys-Thr-NH2
(Mw: 947)

Starting from 0.47 g (0.25 mmol) of benzhydrylamine hydrochloride resin (0.54 milliequivalent/g) the peptide is synthetized, cyclized and purified as described in Example 1 to gave the final product in a yield of 80.5 mg (34 %). The physical characteristics are summarized in Table V, the biological effects already have been shown in Tables I and II.

### Example 3

Preparation of D-Phe-Cys-Tyr-D-Trp-Lys-Leu-Cys-Thr-NH2
(Mw: 1060)

Starting from 0.47 g (0.25 mmol) of benzhydrylamine hydrochloride resin (0.54 milliequivalent/g) the aimed peptide is synthetized, cyclized and purified as described in Example 1 to obtain 106 mg (40 %) of final product. The physical characteristics of this compound are summarized in Table V, the results of biological assays already have been shown in Tables II, III, IV and V.

### Example 4

Preparation of D-Phe-Cys-Tyr-β-Asp(indolinyl)-Lys-
-Leu-Cys-Thr-NH2 (Mw: 1076)

Starting from 0.47 g (0.25 mmol) of benzhydrylamine hydrochloride resin the aimed peptide is synthetized, cyclized and purified as described in Example 1 to give 94 mg (35.5 %) of final product. The physical characteristics of this compound are summarized in Table V, the results of biological assays are shown in Tables I, II, III and IV.

### Example 5

### Preparation of D-Phe-Tyr-Tyr-Aa-Lys-Val-Phe-Trp-NH₂ (Mw: 1192)

Starting from 0.47 g (0.25 mmol) of benzhydrylamine hydrochloride resin the peptide is synthetized analogously as described in Example 1. The D-Phe-Tyr(2-Br-Z)-Tyr(2-Br-Z)-Aa-Lys(2-Cl-Z)-Val-Phe-Trp peptide resin obtained after the last step is suspended in 3 ml of anisole, 30 ml of gaseous HF are condensed onto the mixture which is then stirred at 0 °C for 45 minutes. After removing the gaseous HF the residue is suspended in 200 ml of abs. ether, stirred for 15 minutes and after filtration the precipitate is washed with ether. Thereafter, the peptide is dissolved from the precipitate with 50 % by volume acetic acid, the solution is evaporated and the thus-obtained D-Phe-Tyr-Tyr-Aa-Lys-Val-Phe-Trp-NH₂ heptapeptide amide is purified by preparative HPLC. Eluent A: 0.25 N TEAP, pH 2.25; eluent B: mixture or 70 % of acetonitrile and 30 % of eluent A. The separation is carried out on the column mentioned in Example 1 by using a mixture containing 30 % of eluent B and 70 % of eluent A. The pure product is desalinized as described in Example 1 to obtain 89 mg (30 %) of final product. The physical characteristics of this compound are summarized in Table V, the biological effects already have been shown in Tables I and II.

### Example 6

### Preparation of Ac-Sar-Ala-Tyr-Tyr-Aa-Lys-Val-Phe-Trp-NH₂ (Mw: 1217)

The aimed peptide is prepared by using the process of Example 5 but an Ac-Sar group is coupled to the peptide resin in the last cycle. The cleavage of the product from the resin and the purification are also carried out as described in Example 6 to obtain 85 mg (28 %) of final product. The physical characteristics of the compound are summarized in Table V whereas its biological effects already have been shown in Tables I, II and III.

### Example 7

### Preparation of Pop-Tyr-Tyr-Aa-Lys-Val-Phe-Trp-NH₂ (Mw: 1194)

This peptide is synthetized as described in Example 1, except that in the 11th step Bop reagent is used in an amount equal to the amino acid as activating agent for coupling the amino acid in the presence of an excess of DIPEA. After cleavage by HE and purification by chromatography, the final product is obtained as described in Example 5 with a yield of 105 mg (35 %). The physical characteristics of the compound are summarized in Table V.

### Example 8

### Preparation of Aa-Cys(Acm)-Tyr-D-Trp-Lys-Val-Cys(Acm)-Trp-NH₂ (Mw: 1283)

After swelling 0.47 g (0.25 mmol) of benzhydrylamine hydrochloride resin in methylene chloride for 90 minutes, the following cycle is repeated after each acylation carried out with the Fmoc-derivatives of the suitably protected amino acids the percentages being by volume:

| Step | Reagents, procedure | Time of stirring (minute) |
|---|---|---|
| 1 | DMF; 3 washings | 2 |
| 2 | Mixture containing 20 % of piperidine and 80 % of DMF; cleavage | 2 |
| 3 | Mixture containing 20 % of piperidine and 80 % of DMF; cleavage | 10 |
| 4 | DMF; 5 washings | 2 |
| 5 | Symmetric anhydride⁺ prepared from 3 equivalents of Fmoc-amino acid or Opfp ester; coupling | 60 |
| 6 | DMF; 3 washings | 2 |

| | | |
|---|---|---|
| + = Preparation of the symmetric anhydride: 3 equivalents of Fmoc-amino acid are dissolved in a mixture of CH₂Cl₂ and DMF. After adding 1.5 equivalents of DCC the mixture is stirred at room temperature for 15 minutes. The DCU precipitate is filtered off, the solution is evaporated and the residue dissolved in DMF is used for coupling. | | |

A ninhydrin test is made after each step. When a positive result is obtained, the cycle is repeated from the 5th step.

The Aa-Cys(Acm)-Tyr(tBu)-D-Trp-Lys(Boc)-Val-Cys(Acm)-Trp peptide resin obtained after the last step is treated with HF, purified and desalinized as described in Example 5 to obtain 147 mg (46 %) of final product. The physical characteristics of the compound are summarized in Table V.

### Examples 9 to 14

The compounds of the following formulae, listed hereinafter, are prepared by using the process described in Examples 1 to 4.

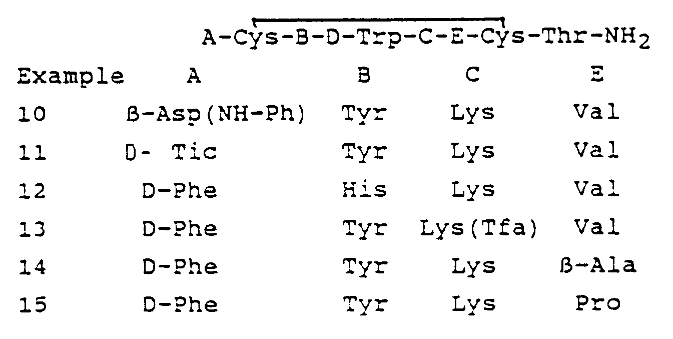

The physical characteristics of the above compounds are summarized in Table V, the results of biological assays already have been shown in Tables I, II and V.

### Examples 15 and 16

Compounds of the following formulae are prepared as described in Example 6:

| A-B-Tyr-Aa-Lys-Val-Phe-Trp-NH₂ | | |
|---|---|---|
| Example | A | B |
| 15 | Ac-Sar | Tyr |
| 16 | D-Phe | Ala |

The physical characteristics of the above compounds are summarized in Table V, the results of biological assays already have been shown in Table I.

### Examples 17 and 18

The following compounds are prepared by using the process described in Example 1: A-Cys-Tyr-Aa-Lys-Val-Cys-Trp-NH2
In Example 17 "A" stands for a D-Phe group, in Example 18 for a D-2-naphthyl-alanyl group. The physical data of the compounds are summarized in Table V.

### Example 19

The following compound is prepared by using the process described in Example 1: Aa-Cys-Tyr-D-Trp-Lys-Val-Cys-Trp-NH2
The physical data of the compounds are summarized in Table V.

### Example 20

D-Phe-Cys-Tyr-Asu-Lys-Val-Cys-Thr-NH2

The compound is prepared by using the process described in Example 1 with the exception that after coupling the Lys residue the neutralization is carried out with 5 % by weight diisopropyl-ethylamine in dichloromethane, instead of using TEA. The side-chain protecting group (fluorenylmethyl ester) of Asp is removed from the protected peptide resin by stirring it for 2x20 minutes in a 1:1 mixture of DMF and piperidine. The formation of Asp-β-pentafluorophenyl ester is carried out by stirring the suspension of the peptide resin for 30 minutes in DMF with an excess of pentafluorophenol and diisopropyl carbodiimide. The peptide resin is filtered and washed with DMF. The aspartimide derivative is formed from the pentafluorophenyl ester derivative by stirring the peptide resin in DMF. The formation of the aspartimide ring is followed by measuring the released pentafluorophenol. The UV absorption of a small amount of the filtered reaction mixture is measured at 278 nm using DMF as control. The cleavage of the remaining protecting groups, the cleavage of the peptide from the resin, the formation of the disulfide bridge and the purification of the crude product are carried out using the process described in Example 1. The physical data of the compound are summarized in Table V.

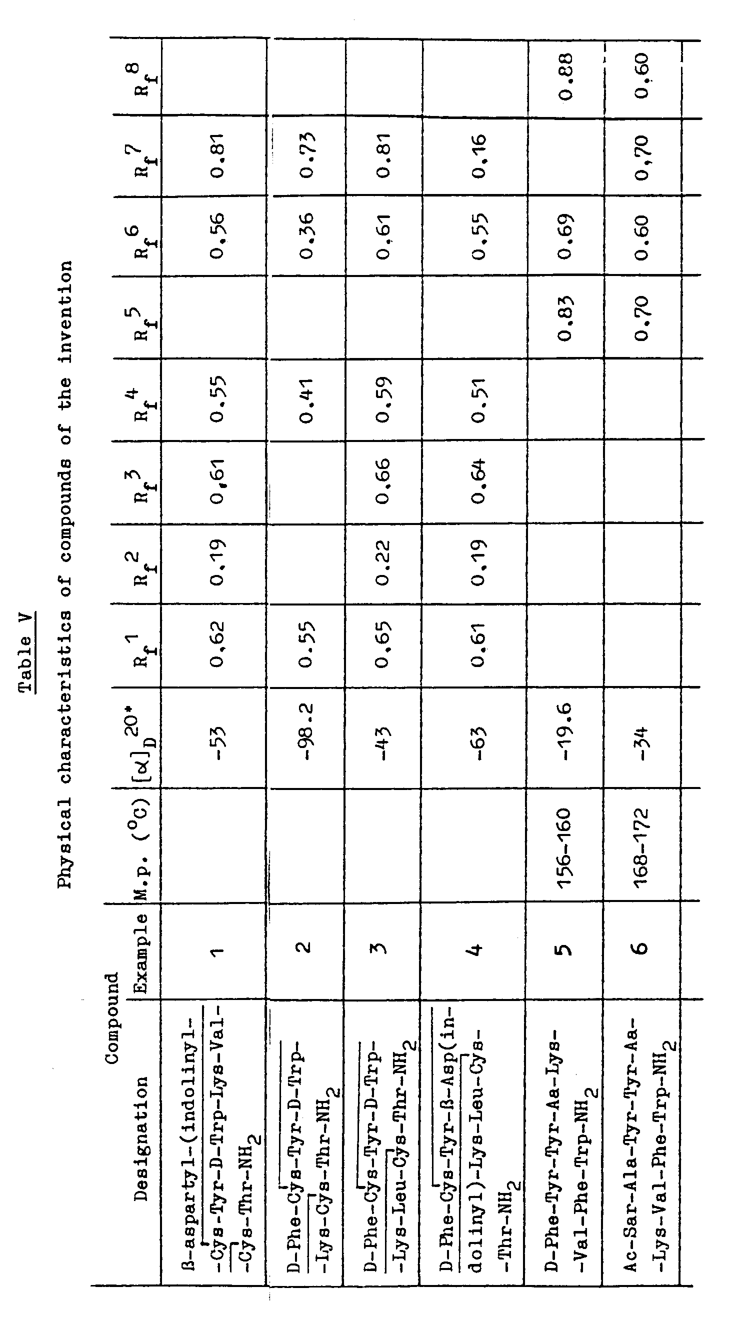

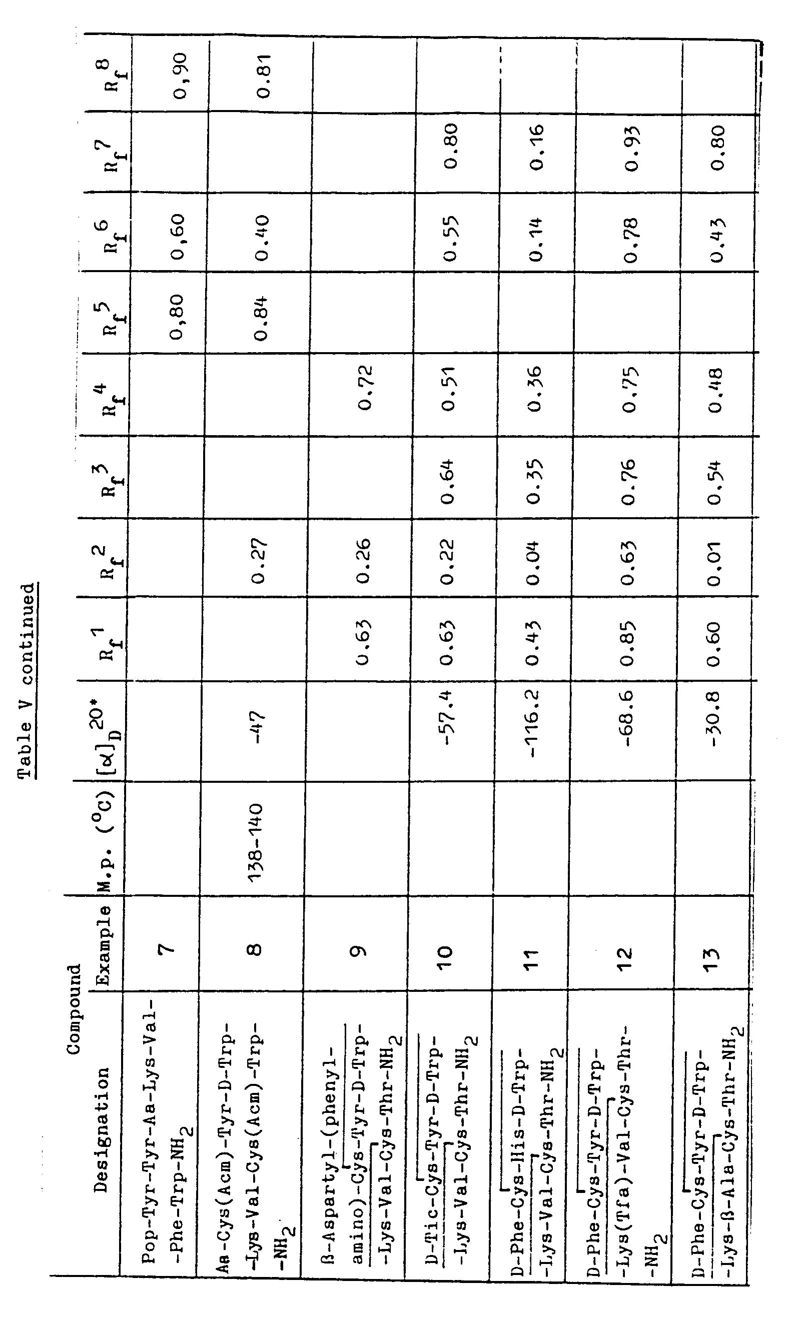

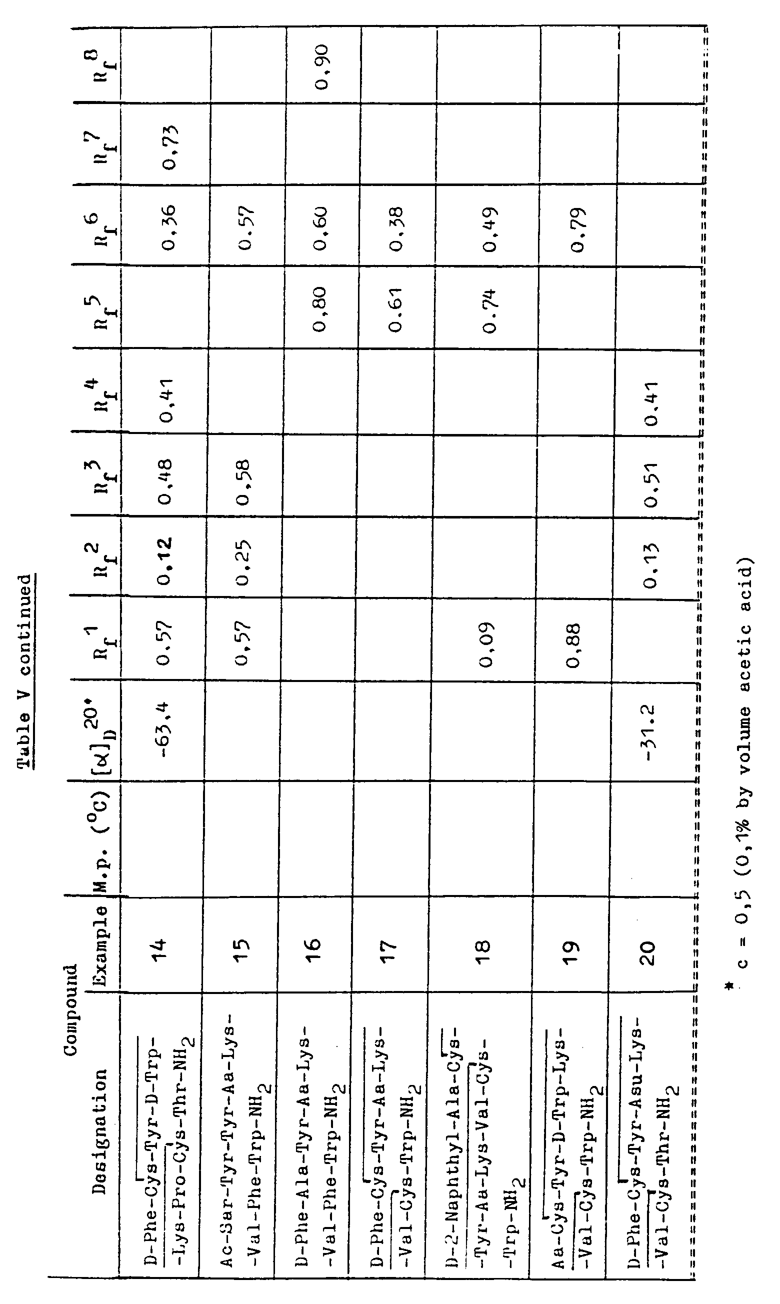

## Claims (Claims for the following Contracting State(s): AT, BE, CH, DE, DK, FR, GB, IT, LI, LU, NL, PT, SE)

1. Octapeptide or heptapeptide amide derivatives of the general formula 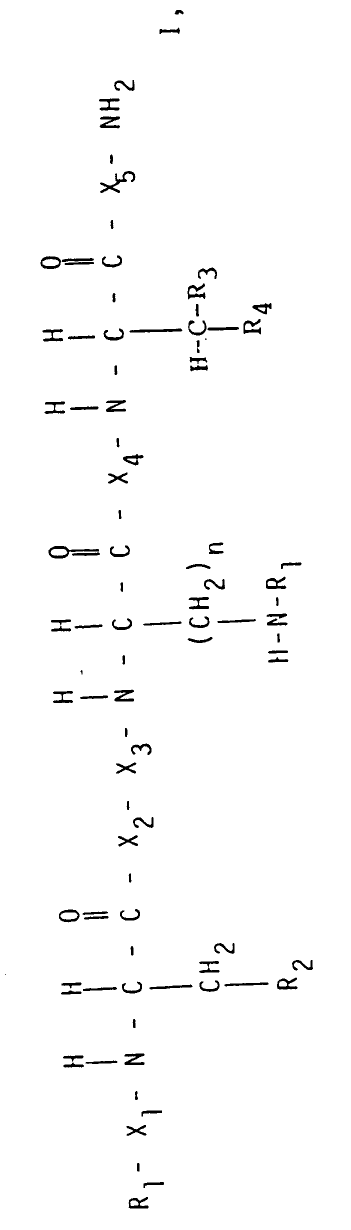 wherein
X₁ stands for an aromatic D-amino acid rest or a derivative thereof ring-substituted by halogen and/or hydroxyl; or a D-phenylglycyl, p-hydroxyphenylglycyl, o-aminobenzoyl, m-aminobenzoyl, D-tetrahydroisoquinolylcarbonyl or sarcosylalanyl; or a 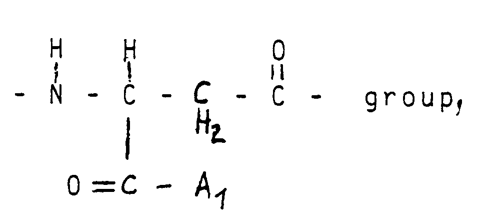 in which latter
A₁ means the rest of an aromatic, heterocyclic or aliphatic amine having a primary or secondary amino group
or means a primary or secondary aromatic, heterocyclic or aliphatic amino group or means an alkoxy, aryloxy or aralkyloxy group;
X₂ represents an aromatic amino acid rest or a derivative thereof ring-substituted by halogen and/or hydroxyl; or a histidyl group;
X₃ means a D-tryptophyl, o-aminobenzoyl, m-aminobenzoyl, aspartyl, D-aspartyl, β-aspartyl, β-D-aspartyl or aminosuccinyl group; or a 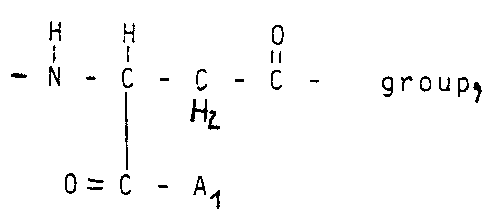 in which latter
A₁ is as defined above;
X₄ stands for an amino acid rest bearing an alkyl or aralkyl side chain, or a derivative thereof substituted by a hydroxyl or methyl group in β-position; or a prolyl or β-alanyl group; or a valence bond;
X₅ means a heterocyclic amino acid rest or a derivative thereof ring-substituted by halogen and/or hydroxyl; or a threonyl group;
R₁ represents hydrogen or a 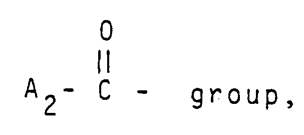 in which latter
A₂ means hydrogen or a C₁₋₄ alkyl group, optionally substituted by halogen;
R₂ means hydrogen, -S- or a -S-acetamidomethyl, phenyl or p-hydroxyphenyl group;
R₃ stands for hydrogen or hydroxyl;
R₄ means a phenyl or p-hydroxyphenyl group; or -S- or a -S-acetamidomethyl group when the meaning of R₂ is the same group; or a methyl group when R₃ is hydroxyl, in case of R₂ and R₄ each being -S- one bond each of them are united to a sole bond representing a bridge between R₂ and R₄ and
n is 1, 2, 3 or 4,
with the provisos that
a) if
X₁ stands for D-β-Nal or D-Phe, optionally substituted by halogen or a hydroxy group,
X₂ means Tyr or Phe
X₃ represents D-Trp,
X₅ stands for Trp or Thr and
R₂ and R₄ each are -S-, one bond each of them being united to a sole bond representing a bridge between R₂ and R₄
X₄ is different from Val, Thr or Ser,
b) if
X₄ stands for Val or Thr or Ser and
X₁ means an aromatic D-amino acid residue or a derivative thereof ring-substituted by halogen or hydroxy
X₂ represents an aromatic amino acid rest or a derivative ring-substituted by halogen or hydroxy
at least one of the following conditions is fulfilled
α)
X₃ is different from D-Trp
or
β)
R₂ and R₄ together have a meaning different from a - S - S - group (disulfide bridge),
and acid addition salts thereof.

2. Octapeptide or heptapeptide amide derivatives according to claim 1, characterized in that the D-amino acid rest which may be represented by X₁ is a D-phenylalanyl, D-naphthylalanyl or D-tryptophyl rest and/or the amino acid rest which may be represented by X₂ is a tyrosyl rest and/or the halogen by which the rest of the D-amino acid derivative which may be represented by X₁ and/or the rest of the amino acid derivative which may be represented by X₂ may be ring-substituted is iodine, particularly preferably the rest of the D-amino acid derivative which may be represented by X₁ being a D-diiodtyrosyl rest and/or the rest of the amino acid derivative which may be represented by X₂ being a diiodtyrosyl rest.

3. Octapeptide or heptapeptide amide derivatives according to claim 1, characterized in that the rest of the amine which may be represented by A₁ is an indolinyl or phenylamino rest, the alkoxy group which may be represented by A₁ is such having from 1 to 4 carbon atom(s), the aryloxy group which may be represented by A₁ is a phenyloxy group or the aralkyloxy group which may be represented by A₁ is such having from 1 to 4 carbon atom(s) in the alkyl part and having as aryl part a phenyl part.

4. Octapeptide or heptapeptide amide derivatives according to claims 1 to 3, characterized in that the alkyl side chain of the amino acid rest or its derivative which may be represented by X₄ is such having from 1 to 4 carbon atom(s) or the aralkyl side chain of the amino acid rest or its derivative which may be represented by X₄ is such having from 1 to 4 carbon atom(s) in the alkyl part and having as aryl part a phenyl part, particularly the amino acid rest which may be represented by X₄ being a leucyl, valyl, isoleucyl, norvalyl, norleucyl alanyl or seryl rest and/or the aromatic amino acid rest which may be represented by X₅ is a tryptophyl or phenylalanyl rest, or the halogen by which the rest of the derivative of the aromatic amino acid which may be represented by X₅ may be substituted is iodine, particularly preferably the rest of the derivative of the aromatic amino acid which may be represented by X₅ being a tyrosyl or diiodtyrosyl rest, and/or the C₁₋₄ alkyl group which may be represented by A₂ is such having 1 or 2 carbon atoms or the halogen by which the C₁₋₄ alkyl group which may be represented by A₂ may be substituted is fluorine and/or n is 3 or 4.

5. A compound as claimed in claims 1 to 4, selected from the group consisting of 
β-aspartyl-(indolinyl)-Cys-
-Tyr-D-Trp-Lys-Val-Cys-Thr-NH2,
D-Phe-Cys-Tyr-D-Trp-Lys-
-Cys-Thr-NH2, D-Phe-Cys-Tyr-D-Trp-Lys-Leu-Cys-Thr-
-NH2, D-Phe-Cys-Tyr-β-Asp(indolinyl)-Lys-Leu-Cys-
-Thr-NH2,
 D-Phe-Tyr-Tyr-Aa-Lys-Val-Phe-Trp-NH₂, Ac-Sar-Ala-Tyr-Tyr-Aa-Lys-Val-Phe-Trp-NH₂, Pop-Tyr-Tyr-Aa-Lys-Val-Phe-Trp-NH₂ and Aa-Cys(Acm)-Tyr-D-Trp-Lys-Val-Cys(Acm)-Trp-NH₂.

6. A process for the preparation or the compounds according to claims 1 to 5, characterized in that using the method of the solid-phase peptide synthesis, the protected amino acids are stepwise condensed onto the solid resin carrier through carbodiimide or active ester coupling in the corresponding succession, then the final product is removed from the solid carrier by acidic or alkaline cleavage and the protective groups are removed from the amino acids simultaneously with or before or after cleavage from the resin and, if desired, the octapeptide or heptapeptide amide of the general formula I thus obtained is converted to an acid addition salt by reacting it with an acid and/or, if desired, the free base is liberated from the acid addition salt obtained by reacting it with an alkali and, if desired, the latter is converted to another acid addition salt by reacting it with an acid.

7. A process as claimed in claim 6, characterized in that a benzhydrylamine resin or a chloromethylated polystyrene resin is used as solid resin carrier.

8. A process as claimed in claim 6 or 7, characterized in that the final product containing protective groups is splitted off by hydrogen fluoride and/or ammonolysis.

9. Medicaments characterized in that they contain as active ingredient(s) 1 or more compound(s) as claimed in claims 1 to 8, suitably in admixture with 1 or more carrier(s) and/or additive(s) commonly used in the pharmaceutical technique.

10. The use of the compounds according to claims 1 to 5 for preparing medicaments, particularly for treating mammals, including man, suffering from tumour disease and/or for inhibiting their tyrosine kinase activity.

## Claims (Claims for the following Contracting State(s): ES, GR)

1. A process for the preparation of octapeptide or heptapeptide amide derivatives of the general formula 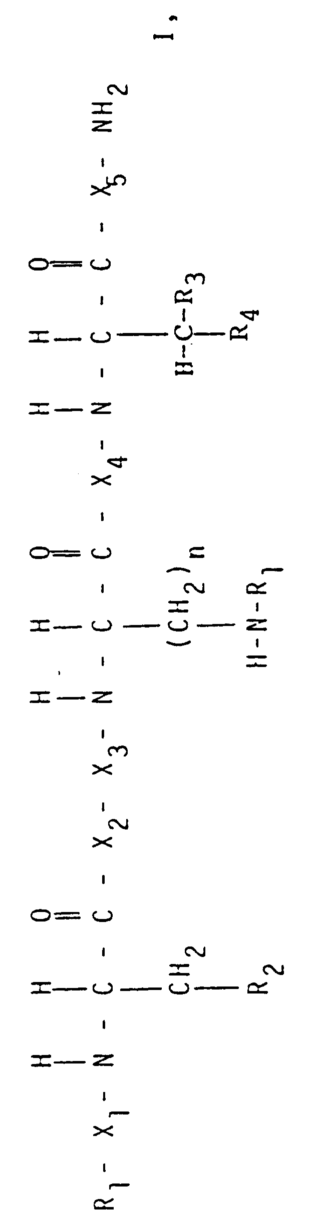 wherein
X₁ stands for an aromatic D-amino acid rest or a derivative thereof ring-substituted by halogen and/or hydroxyl; or a D-phenylglycyl, p-hydroxyphenylglycyl, o-aminobenzoyl, m-aminobenzoyl, D-tetrahydroisoquinolylcarbonyl or sarcosylalanyl; or a 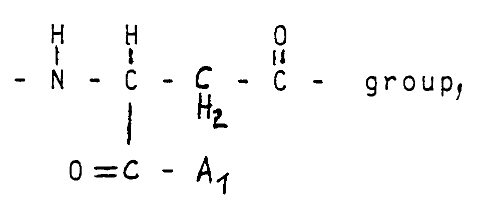 in which latter
A₁ means the rest of an aromatic, heterocyclic or aliphatic amine having a primary or secondary amino group
or means a primary or secondary aromatic, heterocyclic or aliphatic amino group or means an alkoxy, aryloxy or aralkyloxy group;
X₂ represents an aromatic amino acid rest or a derivative thereof ring-substituted by halogen and/or hydroxyl; or a histidyl group;
X₃ means a D-tryptophyl, o-aminobenzoyl, m-aminobenzoyl, aspartyl, D-aspartyl, β-aspartyl, β-D-aspartyl or aminosuccinyl group; or a 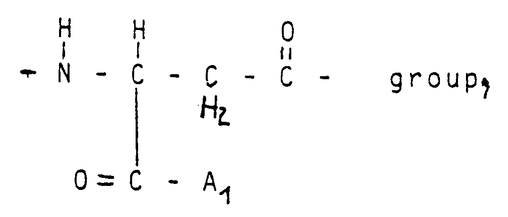 in which latter
A₁ is as defined above;
X₄ stands for an amino acid rest bearing an alkyl or aralkyl side chain, or a derivative thereof substituted by a hydroxyl or methyl group in β-position; or a prolyl or β-alanyl group; or a valence bond;
X₅ means an aromatic amino acid rest or a derivative thereof ring-substituted by halogen and/or hydroxyl; or a threonyl group;
R₁ represents hydrogen or a 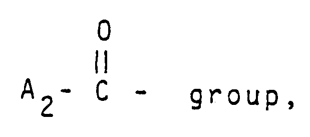 in which latter
A₂ means hydrogen or a C₁₋₄ alkyl group, optionally substituted by halogen;
R₂ means hydrogen, -S- or a -S-acetamidomethyl, phenyl or p-hydroxyphenyl group;
R₃ stands for hydrogen or hydroxyl;
R₄ means a phenyl or p-hydroxyphenyl group; or -S- or a -S-acotamidomethyl group when the meaning of R₂ is the same group; or a methyl group when R₃ is hydroxyl, in case of R₂ and R₄ each being -S- one bond each of them are united to a sole bond representing a bridge between R₂ and R₄
and
n is 1, 2, 3 or 4,
with the provisos that
a) if
X₁ stands for D-β-Nal or D-Phe, optionally substituted halogen or a hydroxy group,
X₂ means Tyr or Phe
X₃ represents D-Trp,
X₅ stands for Trp or Thr and
R₂ and R₄ each are -S-, one bond each of them being united to a sole bond representing a bridge between R₂ and R₄
X₄ is different from Val, Thr or Ser,
b) if
X₄ stands for Val or Thr or Ser and
X₁ means an aromatic D-amino acid residue or a derivative thereof ring-substituted by halogen or hydroxy
X₂ represents an aromatic amino acid rest or a derivative ring-substituted by halogen or hydroxy
at least one of the following conditions is fulfilled
α)
X₃ is different from D-Trp
or
β)
R₂ and R₄ together have a meaning different from a - S - S - group (disulfide bridge),
and acid addition salts thereof. characterized in that using the method of the solid-phase peptide synthesis, the protected amino acids are stepwise condensed onto the solid resin carrier through carbodiimide or active ester coupling in the corresponding succession, then the final product is removed from the solid carrier by acidic or alkaline cleavage and the protective groups are removed from the amino acids simultaneously with or before or after cleavage from the resin and, if desired, the octapeptide or heptapeptide amide of the general formula I thus obtained is converted to an acid addition salt by reacting it with an acid and/or, if desired, the free base is liberated from the acid addition salt obtained by reacting it with an alkali and, if desired, the latter is converted to another acid addition salt by reacting it with an acid.

2. A process as claimed in claim 1, characterized in that a benzhydrylamine resin or a chloromethylated polystyrene resin is used as solid resin carrier.

3. A process as claimed in claim 1 or 2, characterized in that the final product containing protective groups is splitted off by hydrogen fluoride and/or ammonolysis.

4. A process as claimed in claims 1 to 3, characterized in that octapeptide or heptapeptide amide derivatives are prepared in which the D-amino acid rest which may be represented by X₁ is a D-phenylalanyl, D-naphthylalanyl or D-tryptophyl rest and/or the amino acid rest which may be represented by X₂ is a tyrosyl rest and/or the halogen by which the rest of the D-amino acid derivative which may be represented by X₁ and/or the rest of the amino acid derivative which may be represented by X₂ may be ring-substituted is iodine, particularly preferably the rest of the D-amino acid derivative which may be represented by X₁ being a D-diiodtyrosyl rest and/or the rest of the amino acid derivative which may be represented by X₂ being a diiodtyrosyl rest.

5. A process as claimed in claims 1 to 3, characterized in that octapeptide or heptapeptide amide derivatives are prepared in which the rest of the amine which may be represented by A₁ is an indolinyl or phenylamino rest, the alkoxy group which may be represented by A₁ is such having from 1 to 4 carbon atom(s), the aryloxy group which may be represented by A₁ is a phenyloxy group or the aralkyloxy group which may be represented by A₁ is such having from 1 to 4 carbon atom(s) in the alkyl part and having as aryl part a phenyl part.

6. A process as claimed in claims 1 to 3, characterized in that octapeptide or heptapeptide amide derivatives are prepared in which the alkyl side chain of the amino acid rest or its derivative which may be represented by X₄ is such having from 1 to 4 carbon atom(s) or the aralkyl side chain of the amino acid rest or its derivative which may be represented by X₄ is such having from 1 to 4 carbon atom(s) in the alkyl part and having as aryl part a phenyl part, particularly the amino acid rest which may be represented by X₄ being a leucyl, valyl, isoleucyl, norvalyl, norleucyl, alanyl or seryl rest and/or the aromatic amino acid rest which may be represented by X₅ is a tryptophyl or phenylalanyl rest, or the halogen by which the rest of the derivative of the aromatic amino acid which may be represented by X₅ may be substituted is iodine, particularly preferably the rest of the derivative or the aromatic amino acid which may be represented by X₅ being a tyrosyl or diiodtyrosyl rest, and/or the C₁₋₄ alkyl group which may be represented by A₂ is such having 1 or 2 carbon atoms or the halogen by which the C₁₋₄ alkyl group which may be represented by A₂ may be substituted is fluorine and/or n is 3 or 4.

7. A process as claimed in claims 1 to 3, characterized in that octapeptide or heptapeptide amide derivatives are prepared which are selected from the group consisting of 
β-aspartyl-(indolinyl)-Cys-
-Tyr-D-Trp-Lys-Val-Cys-Thr-NH2,
D-Phe-Cys-Tyr-D-Trp-Lys-
-Cys-Thr-NH2, D-Phe-Cys-Tyr-D-Trp-Lys-Leu-Cys-Thr-
-NH2, D-Phe-Cys-Tyr-β-Asp(indolinyl)-Lys-Leu-Cys-
-Thr-NH2,
 D-Phe-Tyr-Tyr-Aa-Lys-Val-Phe-Trp-NH₂, Ac-Sar-Ala-Tyr-Tyr-Aa-Lys-Val-Phe-Trp-NH₂, Pop-Tyr-Tyr-Aa-Lys-Val-Phe-Trp-NH₂, Aa-Cys(Acm)-Tyr-D-Trp-Lys-Val-Cys(Acm)-Trp-NH₂.

8. A process for preparing medicaments characterized by mixing as active ingredient(s) 1 or more compound(s) prepared by the process according to claims 1 to 7, with 1 or more carrier(s) and/or additive(s) commonly used in the pharmaceutical technique in a manner knwon per se.

## Patentansprüche (Patentansprüche für folgende(n) Vertragsstaat(en): AT, BE, CH, DE, DK, FR, GB, IT, LI, LU, NL, PT, SE)

1. Octapeptid- oder Heptapeptidamidderivate der allgemeinen Formel 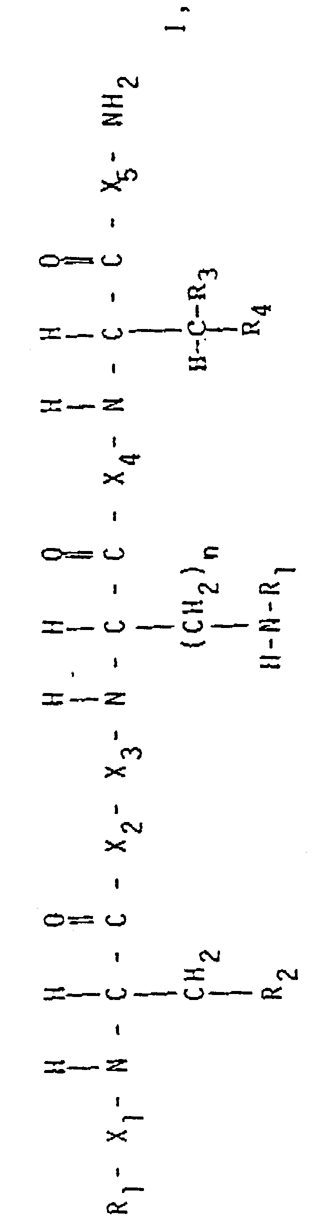 worin
X₁ für einen aromatischen D-Aminosäurerest oder ein Derivat davon, ringsubstituiert durch Halogen und/oder Hydroxyl; oder für ein D-Phenylglycyl, p-Hydroxyphenyl-glycyl, o-Aminobenzoyl, m-Aminobenzoyl, D-Tetrahydroisochinolylcarbonyl oder Sarcosylalanyl; oder für eine 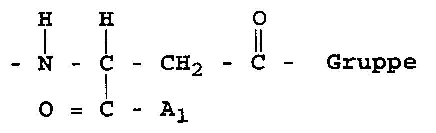
steht, wobei in letzterer
A₁ den Rest eines aromatischen, heterocyclischen oder aliphatischen Amins mit einer primären oder sekundären Aminogruppe,
oder eine primäre oder sekundäre, aromatische, heterocyclische oder aliphatische Aminogruppe, oder eine Alkoxy-, Aryloxy- oder Aralkyloxygruppe bedeutet;
X₂ für einen aromatischen Aminosäurerest oder ein Derivat davon, ringsubstituiert durch Halogen und/oder Hydroxyl; oder für eine Histidylgruppe steht;
X₃ für eine D-Tryptophyl-, o-Aminobenzoyl-, m-Aminobenzoyl-, Aspartyl-, D-Aspartyl-, D-Aspartyl-, β-Aspartyl-, β-D-Aspartyl- oder Aminosuccinylgruppe; oder für eine 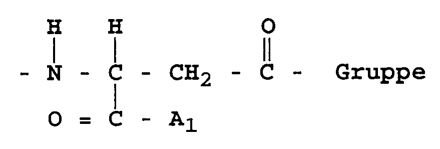
steht, wobei in letzterer
A₁ die obenstehende Bedeutung besitzt;
X₄ für einen eine Alkyl- oder Aralkylseitenkette tragenden Aminosäurerest oder ein Derivat davon, substituiert durch eine Hydroxyl- oder Methylgruppe in β-Position; oder für eine Prolyl- oder β-Alanylgruppe; oder für eine Valenzbindung steht;
X₅ einen heterocyclischen Aminosäurerest oder ein Derivat davon, rinsubstituiert durch Halogen und/oder Hydroxyl; oder eine Threonylgruppe bedeutet;
R₁ für Wasserstoff oder eine 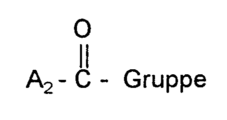 steht,
wobei in letzterer
A₂ für Wasserstoff oder eine C₁₋₄-Alkylgruppe, wahlweise durch Halogen substituiert, steht;
R₂ Wasserstoff, -S- oder eine -S-Acetamidomethyl-, Phenyl- oder p-Hydroxyphenylgruppe bedeutet;
R₃ für Wasserstoff oder Hydroxyl steht;
R₄ eine Phenyl- oder p-Hydroxyphenylgruppe; oder eine -S-Gruppe oder eine -S-Acetamidomethylgruppe, wenn R₂ die gleiche Gruppe bedeutet; oder eine Methylgruppe, wenn R₃ Hydroxyl ist, bedeutet, wobei im Falle, daß R₂ und R₄ jeweils eine -S-Bindung bedeuten, jeweils eine Bindung von diesen zu einer Bindung verbunden ist, was eine Brücke zwischen R₂ und R₄ ergibt
und worin
n 1, 2, 3 oder 4 ist,
mit der Maßgabe, daß wenn
a)
X₁ für D-β-Nal oder D-Phe, wahlweise durch Halogen oder eine Hydroxygruppe substituiert, steht,
X₂ Tyr oder Phe bedeutet,
X₃ D-Trp bedeutet,
X₅ Trp oder Thr bedeutet und
R₂ und R₄ jeweils -S- bedeuten, wobei jeweils eine Bindung von diesen zu einer Bindung verbunden sind, was eine Brücke zwischen R₂ und R₄ ergibt,
X₄ nicht Val, Thr oder Ser ist;
b)
X₄ Val oder Thr oder Ser bedeutet und
X₁ einen aromatischen D-Aminosäurerest oder ein Derivat davon, ringsubstituiert durch Halogen oder Hydroxy, bedeutet,
X₂ für einen aromatischen Aminosäurerest oder ein Derivat, ringsubstituiert durch Halogen oder Hydroxy, steht,
mindestens eine der folgenden Bedingungen erfüllt ist:
α)
X₃ nicht D-Trp ist
oder
β)
R₂ und R₄ zusammen nicht die Bedeutung einer -S-S-Gruppe (Disulfidbrücke) besitzen;
und Säureadditionssalze davon.

2. Octapeptid- oder Heptapeptidamidderivate gemäß Anspruch 1, **dadurch gekennzeichnet,** daß der D-Aminosäurerest, welcher durch X₁ repräsentiert werden kann, ein D-Phenylalanyl-, D-Naphthylalanyl- oder D-Tryptophylrest ist, und/oder der Aminosäurerest, der durch X₂ repräsentiert werden kann, ein Tyrosylrest ist, und/oder das Halogen, durch das der Rest des D-Aminosäurederivates, welches durch X₁ repräsentiert werden kann, und/oder der Rest des Aminosäurederivates, welcher durch X₂ repräsentiert werden kann, ring-substituiert werden kann, Iod ist, wobei besonders bevorzugt der Rest des D-Aminosäurederivates, welcher durch X₁ repräsentiert werden kann, ein D-Diiodtyrosylrest ist, und/oder der Rest des Aminosäurederivates, welcher durch X₂ repräsentiert werden kann, ein Diiodtyrosylrest ist.

3. Octapeptid- oder Heptapeptidamidderivate gemäß Anspruch 1, **dadurch gekennzeichnet,** daß der Rest des Amins, welches durch A₁ repräsentiert werden kann, ein Indolinyl- oder Phenylaminorest ist, die Alkoxygruppe, welche durch A₁ repräsentiert werden kann, eine solche mit 1 bis 4 Kohlenstoffatomen ist, die Aryloxygruppe, welche durch A₁ repräsentiert werden kann, eine Phenyloxygruppe ist, oder die Aralkyloxygruppe, welche durch A₁ repräsentiert werden kann, eine solche mit 1 bis 4 Kohlenstoffatomen in dem Alkylteil und einem Phenylteil als Arylteil ist.

4. Octapeptid- oder Heptapeptidamidderivate gemaß Anspruch 1 bis 3, **dadurch gekennzeichnet,** daß die Alkylseitenkette des Aminosäurerestes oder seines Derivates, welcher durch X₄ repräsentiert werden kann, ein solcher mit 1 bis 4 Kohlenstoffatomen ist, oder die Aralkylseitenkette des Aminosäurerestes oder seines Derivates, welcher durch X₄ repräsentiert werden kann, eine solche mit 1 bis 4 Kohlenstoffatomen im Alkylteil und einem Phenylteil als Arylteil ist, wobei insbesondere der Aminosäurerest, welcher durch X₄ repräsentiert werden kann, ein Leucyl-, Valyl-, Isoleucyl-, Norvalyl-, Norleucyl-, Alanyl- oder Serylrest ist, und/oder der aromatische Aminosäurerest, welcher durch X₅ repräsentiert werden kann, ein Tryptophyl- oder Phenylalanylrest ist,
oder das Halogen, durch das der Rest des Derivats der aromatischen Aminosäure, welche durch X₅ repräsentiert werden kann, substituiert werden kann, Iod ist, wobei besonders bevorzugt der Rest des Derivats der aromatischen Aminosäure, welche durch X₅ repräsentiert werden kann, ein Tyrosyl- oder Diiodtyrosylrest ist, und/oder die C₁₋₄-Alkylgruppe, welche durch A₂ repräsentiert werden kann, eine solche mit 1 oder 2 Kohlenstoffatomen ist, oder das Halogen, durch das die C₁₋₄-Alkylgruppe, welche durch A₂ repräsentiert werden kann, substituiert werden kann, Fluor ist und/oder n 3 oder 4 ist.

5. Verbindung gemäß Anspruch 1 bis 4, gewählt aus der aus 
β-Aspartyl-(Indolinyl)-Cys-Tyr-D-Trp-Lys-Val-Cys-Thr-NH2,
D-Phe-Cys-Tyr-D-Trp-Lys-Cys-Thr-NH2,
D-Phe-Cys-Tyr-D-Trp-Lys-Leu-Cys-Thr-NH2,
D-Phe-Cys-Tyr-β-Asp(Indolinyl)-Lys-Leu-Cys-Thr-NH2,
 D-Phe-Tyr-Tyr-Aa-Lys-Val-Phe-Trp-NH₂,
Ac-Sar-Ala-Tyr-Tyr-Aa-Lys-Val-Phe-Trp-NH₂,
Pop-Tyr-Tyr-Aa-Lys-Val-Phe-Trp-NH₂ und Aa-Cys(Acm)-Tyr-D-Trp-Lys-Val-Cys(Acm)-Trp-NH₂ bestehenden Gruppe.

6. Verfahren zur Herstellung der Verbindungen gemäß Anspruch 1 bis 5, **dadurch gekennzeichnet,** daß das Verfahren der Festphasen-Peptidsynthese angewandt wird, wobei die geschützten Aminosäuren schrittweise auf dem festen Harzträger durch Carbodiimidkopplung oder durch Kopplung mittels aktivem Ester in der entsprechenden Reihenfolge kondensiert werden, dann das Endprodukt von dem festen Träger durch saure oder alkalische Spaltung entfernt wird und die Schutzgruppen von den Aminosäuren gleichzeichtig mit oder vor oder nach der Abspaltung vom Harz entfernt werden, und, falls gewünscht, das derart erhaltene Octapeptid- oder Heptapeptidamid der allgemeinen Formel I zu einem Säureadditionssalz durch Umsetzung mit einer Säure umgewandelt wird, und/oder, falls gewünscht, die freie Base von dem erhaltenen Säureadditionssalz durch Umsetzung mit einer Alkalie freigesetzt wird, und, falls gewünscht, letztere zu einem anderen Säureadditionssalz durch Umsetzung dieser mit einer Säure umgewandelt wird.

7. Verfahren gemäß Anspruch 6, **dadurch gekennzeichnet,** daß ein Benzhydrylaminharz oder ein chlormethyliertes Polystyrolharz als fester Harzträger verwendet wird.

8. Verfahren gemäß Anspruch 6 oder 7, **dadurch gekennzeichnet,** daß das Schutzgruppen enthaltende Endprodukt durch Fluorwasserstoff und/oder Ammonolyse aufgepalten wird.

9. Medikamente, **dadurch gekennzeichnet,** daß sie als aktive Bestandteile 1 oder mehrere Verbindungen gemäß den Ansprüchen 1 bis 8 enthalten, geeigneterweise in Vermischung mit 1 oder mehreren Trägern und/oder Additiven, wie sie üblicherweise in der pharmazeutischen Technik verwendet werden.

10. Verwendung der Verbindungen gemäß den Ansprüchen 1 bis 5 zur Herstellung von Medikamenten, insbesondere zur Behandlung von Säugetieren, einschließlich Menschen, die an Tumorerkrankungen leiden, und/oder zur Inhibierung ihrer Tyrosinkinaseaktivität.

## Patentansprüche (Patentansprüche für folgende(n) Vertragsstaat(en): ES, GR)

1. Verfahren zur Herstellung von Octapeptid- oder Heptapeptidamidderivaten der allgemeinen Formel 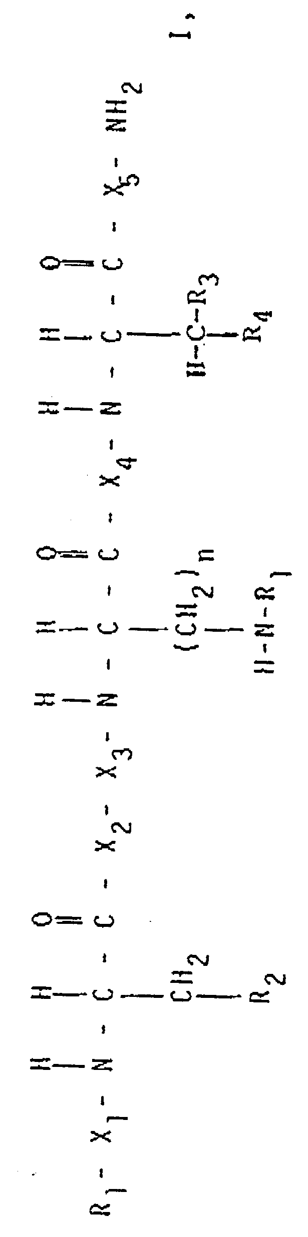 worin
X₁ für einen aromatischen D-Aminosäurerest oder ein Derivat davon, ringsubstituiert durch Halogen und/oder Hydroxyl; oder für ein D-Phenylglycyl, p-Hydroxyphenyl-glycyl, o-Aminobenzoyl, m-Aminobenzoyl, D-Tetrahydroisochinolylcarbonyl oder Sarcosylalanyl; oder für eine 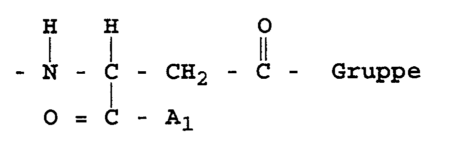
steht, wobei in letzterer
A₁ den Rest eines aromatischen, heterocyclischen oder aliphatischen Amins mit einer primären oder sekundären Aminogruppe,
oder eine primäre oder sekundäre, aromatische, heterocyclische oder aliphatische Aminogruppe, oder eine Alkoxy-, Aryloxy- oder Aralkyloxygruppe bedeutet;
X₂ für einen aromatischen Aminosäurerest oder ein Derivat davon, ringsubstituiert durch Halogen und/oder Hydroxyl; oder für eine Histidylgruppe steht;
X₃ für eine D-Tryptophyl-, o-Aminobenzoyl-, m-Aminobenzoyl-, Aspartyl-, D-Aspartyl-, D-Aspartyl-, β-Aspartyl-, β-D-Aspartyl- oder Aminosuccinylgruppe; oder für eine 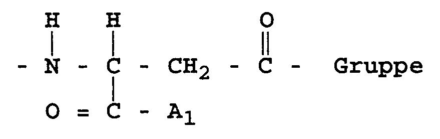
steht, wobei in letzterer
A₁ die obenstehende Bedeutung besitzt;
X₄ für einen eine Alkyl- oder Aralkylseitenkette tragenden Aminosäurerest oder ein Derivat davon, substituiert durch eine Hydroxyl- oder Methylgruppe in β-Position; oder für eine Prolyl- oder β-Alanylgruppe; oder für eine Valenzbindung steht;
X₅ einen heterocyclischen Aminosäurerest oder ein Derivat davon, rinsubstituiert durch Halogen und/oder Hydroxyl; oder eine Threonylgruppe bedeutet;
R₁ für Wasserstoff oder eine 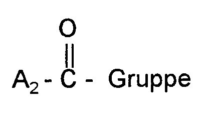 steht,
wobei in letzterer
A₂ für Wasserstoff oder eine C₁₋₄-Alkylgruppe, wahlweise durch Halogen substituiert, steht;
R₂ Wasserstoff, -S- oder eine -S-Acetamidomethyl-, Phenyl- oder p-Hydroxyphenylgruppe bedeutet;
R₃ für Wasserstoff oder Hydroxyl steht;
R₄ eine Phenyl- oder p-Hydroxyphenylgruppe; oder eine -S-Gruppe oder eine -S-Acetamidomethylgruppe, wenn R₂ die gleiche Gruppe bedeutet; oder eine Methylgruppe, wenn R₃ Hydroxyl ist, bedeutet, wobei im Falle, daß R₂ und R₄ jeweils eine -S-Bindung bedeuten, jeweils eine Bindung von diesen zu einer Bindung verbunden ist, was eine Brücke zwischen R₂ und R₄ ergibt
und worin
n 1, 2, 3 oder 4 ist,
mit der Maßgabe, daß wenn
a)
X₁ für D-β-Nal oder D-Phe, wahlweise durch Halogen oder eine Hydroxygruppe substituiert, steht,
X₂ Tyr oder Phe bedeutet,
X₃ D-Trp bedeutet,
X₅ Trp oder Thr bedeutet und
R₂ und R₄ jeweils -S- bedeuten, wobei jeweils eine Bindung von diesen zu einer Bindung verbunden sind, was eine Brücke zwischen R₂ und R₄ ergibt,
X₄ nicht Val, Thr oder Ser ist;
b)
X₄ Val oder Thr oder Ser bedeutet und
X₁ einen aromatischen D-Aminosäurerest oder ein Derivat davon, ringsubstituiert durch Halogen oder Hydroxy, bedeutet,
X₂ für einen aromatischen Aminosäurerest oder ein Derivat, ringsubstituiert durch Halogen oder Hydroxy, steht,
mindestens eine der folgenden Bedingungen erfüllt ist:
α)
X₃ nicht D-Trp ist
oder
β)
R₂ und R₄ zusammen nicht die Bedeutung einer -S-S-Gruppe (Disulfidbrücke) besitzen;
und Säureadditionssalze davon;
**dadurch gekennzeichnet,** daß das Verfahren der Festphasen-Peptidsynthese angewandt wird, wobei die geschützten Aminosäuren schrittweise auf dem festen Harzträger durch Carbodiimidkopplung oder durch Kopplung mittels aktivem Ester in der entsprechenden Reihenfolge kondensiert werden, dann das Endprodukt von dem festen Träger durch saure oder alkalische Spaltung entfernt wird und die Schutzgruppen von den Aminosäuren gleichzeichtig mit oder vor oder nach der Abspaltung vom Harz entfernt werden, und, falls gewünscht, das derart erhaltene Octapeptid- oder Heptapeptidamid der allgemeinen Formel I zu einem Säureadditionssalz durch Umsetzung mit einer Säure umgewandelt wird, und/oder, falls gewünscht, die freie Base von dem erhaltenen Säureadditionssalz durch Umsetzung mit einer Alkalie freigesetzt wird, und, falls gewünscht, letztere zu einem anderen Säureadditionssalz durch Umsetzung dieser mit einer Säure umgewandelt wird.

2. Verfahren gemäß Anspruch 1, **dadurch gekennzeichnet,** daß ein Benzhydrylaminharz oder ein chlormethyliertes Polystyrolharz als fester Harzträger verwendet wird.

3. Verfahren gemäß Anspruch 1 oder 2, **dadurch gekennzeichnet,** daß das Schutzgruppen enthaltende Endprodukt durch Fluorwasserstoff und/oder Ammonolyse aufgepalten wird.

4. Verfahren gemäß den Ansprüchen 1 bis 3, **dadurch gekennzeichnet,** daß Octapeptid- oder Heptapeptidamidderivate hergestellt werden, in denen der D-Aminosäurerest, welcher durch X₁ repräsentiert werden kann, ein D-Phenylalanyl-, D-Naphthylalanyl- oder D-Tryptophylrest ist, und/oder der Aminosäurerest, der durch X₂ repräsentiert werden kann, ein Tyrosylrest ist, und/oder das Halogen, durch das der Rest des D-Aminosäurederivates, welches durch X₁ repräsentiert werden kann, und/oder der Rest des Aminosäurederivates, welcher durch X₂ repräsentiert werden kann, ringsubstituiert werden kann, Iod ist, wobei besonders bevorzugt der Rest des D-Aminosäurederivates, welcher durch X₁ repräsentiert werden kann, ein D-Diiodtyrosylrest ist, und/oder der Rest des Aminosäurederivates, welcher durch X₂ repräsentiert werden kann, ein Diiodtyrosylrest ist.

5. Verfahren gemäß den Ansprüchen 1 bis 3, **dadurch gekennzeichnet,** daß Octapeptid- oder Heptapeptidamidderivate hergestellt werden, in denen der Rest des Amins, welches durch A₁ repräsentiert werden kann, ein Indolinyl- oder Phenylaminorest ist, die Alkoxygruppe, welche durch A₁ repräsentiert werden kann, eine solche mit 1 bis 4 Kohlenstoffatomen ist, die Aryloxygruppe, welche durch A₁ repräsentiert werden kann, eine Phenyloxygruppe ist, oder die Aralkyloxygruppe, welche durch A₁ repräsentiert werden kann, eine solche mit 1 bis 4 Kohlenstoffatomen in dem Alkylteil und einem Phenylteil als Arylteil ist.

6. Verfahren gemäß den Ansprüchen 1 bis 3, **dadurch gekennzeichnet,** daß Octapeptid- oder Heptapeptidamidderivate hergestellt werden, in denen die Alkylseitenkette des Aminosäurerestes oder seines Derivates, welcher durch X₄ repräsentiert werden kann, ein solcher mit 1 bis 4 Kohlenstoffatomen ist, oder die Aralkylseitenkette des Aminosäurerestes oder seines Derivates, welcher durch X₄ repräsentiert werden kann, eine solche mit 1 bis 4 Kohlenstoffatomen im Alkylteil und einem Phenylteil als Arylteil ist, wobei insbesondere der Aminosäurerest, welcher durch X₄ repräsentiert werden kann, ein Leucyl-, Valyl-, Isoleucyl-, Norvalyl-, Norleucyl-, Alanyl- oder Serylrest ist, und/oder der aromatische Aminosäurerest, welcher durch X₅ repräsentiert werden kann, ein Tryptophyl- oder Phenylalanylrest ist,
oder das Halogen, durch das der Rest des Derivats der aromatischen Aminosäure, welche durch X₅ repräsentiert werden kann, substituiert werden kann, Iod ist, wobei besonders bevorzugt der Rest des Derivats der aromatischen Aminosäure, welche durch X₅ repräsentiert werden kann, ein Tyrosyl- oder Diiodtyrosylrest ist, und/oder die C₁₋₄-Alkylgruppe, welche durch A₂ repräsentiert werden kann, eine solche mit 1 oder 2 Kohlenstoffatomen ist, oder das Halogen, durch das die C₁₋₄-Alkylgruppe, welche durch A₂ repräsentiert werden kann, substituiert werden kann, Fluor ist und/oder n 3 oder 4 ist.

7. Verfahren gemäß den Ansprüchen 1 bis 3, **dadurch gekennzeichnet,** daß Octapeptid- oder Heptapeptidamidderivate hergestellt werden, welche gewählt werden aus der aus 
β-Aspartyl-(Indolinyl)-Cys-Tyr-D-Trp-Lys-Val-Cys-Thr-NH2,
D-Phe-Cys-Tyr-D-Trp-Lys-Cys-Thr-NH2,
D-Phe-Cys-Tyr-D-Trp-Lys-Leu-Cys-Thr-NH2,
D-Phe-Cys-Tyr-β-Asp(Indolinyl)-Lys-Leu-Cys-Thr-NH2,
 D-Phe-Tyr-Tyr-Aa-Lys-Val-Phe-Trp-NH₂,
Ac-Sar-Ala-Tyr-Tyr-Aa-Lys-Val-Phe-Trp-NH₂,
Pop-Tyr-Tyr-Aa-Lys-Val-Phe-Trp-NH₂ und Aa-Cys(Acm)-Tyr-D-Trp-Lys-Val-Cys(Acm)-Trp-NH₂ bestehenden Gruppe.

8. Verfahren zur Herstellung von Medikamenten, **dadurch gekennzeichnet,** daß als aktive Bestandteile 1 oder mehrere Verbindungen gemäß den Ansprüchen 1 bis 7 mit 1 oder mehreren Trägern und/oder Additiven, wie sie üblicherweise in der pharmazeutischen Technik verwendet werden, in einer per se bekannten Weise vermischt werden.

## Revendications (Revendications pour l'(les) Etat(s) contractant(s) suivant(s): AT, BE, CH, DE, DK, FR, GB, IT, LI, LU, NL, PT, SE)

1. Dérivés octapeptidyl ou heptapeptidyl amide de formule générale : 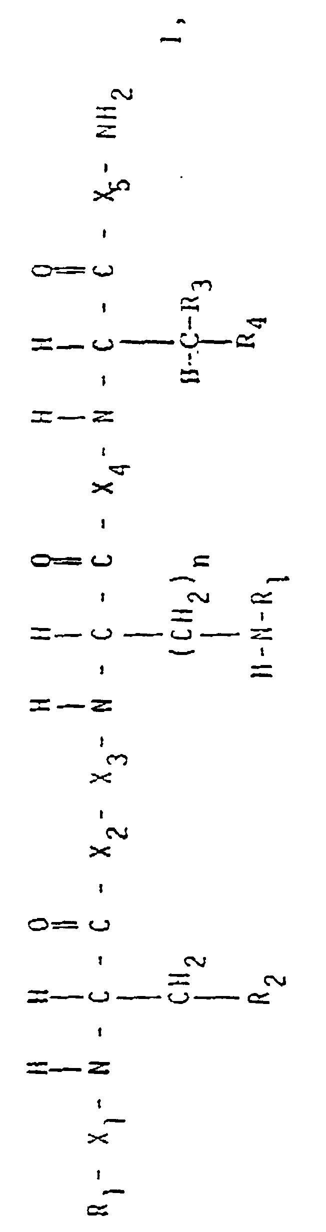 dans laquelle :
- X₁ représente un reste D-aminoacide aromatique ou un dérivé de celui-ci substitué sur le cycle par un halogène et/ou un groupe hydroxyle; ou un groupe D-phénylglycyle, p-hydroxyphénylglycyle, o-aminobenzoyle, m-aminobenzoyle, D-tétrahydroisoquinoléylcarbonyle, ou sarcosyl-analyle; ou un groupe : 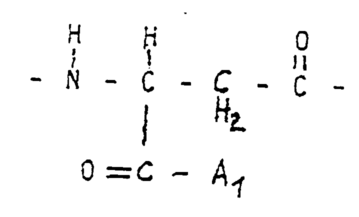 dans lequel :
A₁ représente le reste d'une amine aromatique, hétérocyclique, ou aliphatique comportant un groupe amine primaire ou secondaire, ou
représente un groupe amine aromatique, hétérocyclique, ou aliphatique, primaire ou secondaire,
ou
représente un groupe alcoxy, aryloxy, ou aralkyloxy;
- X₂ représente un reste aminoacide aromatique, ou un dérivé de celui-ci, substitué sur le cycle par un halogène et/ou un groupe hydroxyle; ou un groupe histidyle;
- X₃ représente un groupe D-tryptophyle, o-aminobenzoyle, m-aminobenzoyle, aspartyle, D-aspartyle, β-aspartyle, β-D-aspartyle, ou aminosuccinyle; ou un groupe : 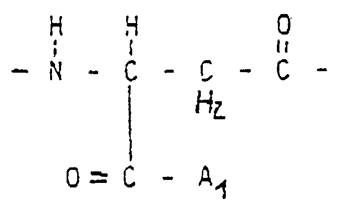 dans lequel :
A₁ est tel que défini ci-dessus;
- X₄ représente un reste aminoacide portant une chaîne latérale alkyle ou aralkyle, ou un dérivé de celui-ci, substitué par un groupe hydroxyle ou méthyle en position β; ou un groupe prolyle ou β-alanyle; ou une liaison de valence;
- X₅ représente un reste aminoacide hétérocyclique, ou un dérivé de celui-ci, substitué sur le cycle par un halogène ou un groupe hydroxyle; ou un groupe thréonyle;
- R₁ représente un atome d'hydrogène ou un groupe : 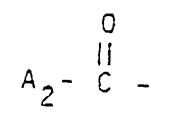 dans lequel :
A₂ représente un atome d'hydrogène ou un groupe alkyle C₁₋₄, éventuellement substitué par un halogène;
- R₂ représente un atome d'hydrogène, un groupe -S-, ou un groupe -S-acétamidométhyle, phényle, ou p-hydroxyphényle;
- R₃ représente un atome d'hydrogène ou un groupe hydroxyle;
- R₄ représente un groupe phényle ou p-hydroxyphényle; ou un groupe -S-, ou -S-acétamidométhyle lorsque R₂ représente le même groupe; ou un groupe méthyle lorsque R₃ est un groupe hydroxyle, dans le cas où R₂ et R₄ sont chacun un groupe -S-, et une liaison de l'un est unie à une liaison de l'autre en une liaison unique représentant un pont entre R₂ et R₄, et
- n est égal à 1, 2, 3, ou 4
à condition que :
a) si
X₁ représente un groupe D-β-Nal ou D-Phe, éventuellement substitué par un halogène ou un groupe hydroxy,
X₂ représente un groupe Tyr ou Phe
X₃ représente un groupe D-Trp
X₅ représente un groupe Trp ou Thr, et
R₂ et R₄ représentent chacun un groupe -S-, une liaison de l'un étant unie à une liaison de l'autre en une liaison unique représentant un pont entre R₂ et R₄
X₄ est un groupe différent de Val, Thr, ou Ser,
b) si
X₄ représente un groupe Val, ou Thr, ou Ser, et
X₁ représente un résidu D-aminoacide aromatique, ou un dérivé de celui-ci, substitué sur le cycle par un halogène ou un groupe hydroxy,
X₂ représente un reste aminoacide aromatique, ou un dérivé de celui-ci, substitué sur le cycle par un halogène ou un groupe hydroxy,
au moins une des conditions suivantes soit remplie :
α) X₃ est un groupe différent de D-Trp, ou
β) R₂ et R₄ ensemble, représentent un groupe différent d'un groupe -S-S- (pont disulfure),
et les sels d'addition d'acide de ceux-ci.

2. Dérivés octapeptidyl ou heptapeptidyl amide selon la revendication 1, caractérisés en ce que le reste D-aminoacide pouvant être représenté par X₁, est un reste D-phénylalanyle, D-naphtylalanyle, ou D-tryptophyle, et/ou le reste aminoacide pouvant être représenté par X₂ est un reste tyrosyle, et/ou l'halogène par lequel le reste du dérivé aminoacide pouvant être représenté par X₁, et/ou le reste du dérivé aminoacide povant être représenté par X₂, peuvent être substitués sur le cycle, est l'iode, par préférence particulière, le reste de dérivé D-aminoacide pouvant être représenté par X₁, étant un reste D-diiodotyrosyle, et/ou le reste, de dérivé aminoacide pouvant peut être représenté par X₂, étant un reste diiodotyrosyle.

3. Dérivés octapeptidyl ou heptapeptidyl amide selon la revendication 1, caractérisés en ce que le reste de l'amine pouvant être représenté par A₁, est un reste indolinyle ou phénylamino, le groupe alcoxy pouvant être représenté par A₁, est tel qu'il comporte 1 à 4 atome(s) de carbone, le groupe aryloxy pouvant être représenté par A₁, est un groupe phényloxy, ou le groupe aralkyloxy pouvant être représenté par A₁, est tel qu'il comporte 1 à 4 atome(s) de carbone dans la partie alkyle, et qu'il a une partie phényle en tant que partie aryle.

4. Dérivés octapeptidyl ou heptapeptidyl amide selon les revendications 1 à 3, caractérisés en ce que la chaîne latérale alkyle du reste aminoacide ou de son dérivé, pouvant être représentée par X₄ est telle qu'elle comporte 1 à 4 atome(s) de carbone, ou que la chaîne latérale aralkyle du reste aminoacide ou de son dérivé, pouvant être représentée par X₄, est telle qu'elle comporte 1 à 4 atome(s) de carbone dans la partie alkyle, et qu'elle a une partie phényle en tant que partie aryle, et en particulier, le reste aminoacide pouvant être représenté par X₄ est un reste leucyle, valyle, isoleucyle, norvalyle, norleucyle alanyle ou séryle, et/ou le reste aminoacide pouvant être représenté par X₅ est un reste tryptophyle ou phénylalanyle, ou l'halogéne par lequel le reste du dérivé aminoacide aromatique pouvant être représenté par X₅, peut être substitué, est l'iode, et, par préférence particulière, le reste de dérivé aminoacide aromatique pouvant être représenté par X₅, est un reste tyrosyle ou diiodotyrosyle, et/ou le groupe alkyle C₁₋₄ pouvant être représenté par A₂, est tel qu'il comporte 1 à 2 atomes de carbone, ou l'halogène par lequel le groupe alkyle C₁₋₄, pouvant être représenté par A₂, peut être substitué, est le fluor, et/ou n est égal à 3 ou à 4.

5. Composé selon les revendications 1 à 4, choisi au sein du groupe comprenant les groupes : 
- β-aspartyl-(indolinyl)-Cys-Tyr-D-Trp-Lys-Val-Cys-Thr-NH2

- D-Phe-Cys-Tyr-D-Trp-Lys-Cys-Thr-NH2

- D-Phe-Cys-Tyr-D-Trp-Lys-Leu-Cys-Thr-NH2

- D-Phe-Cys-Tyr-β-Asp(indolinyl)-Lys-Leu-Cys-Thr-NH2
 - D-Phe-Tyr-Tyr-Aa-Lys-Val-Phe-Trp-NH₂
- Ac-Sar-Ala-Tyr-Tyr-Aa-Lys-Val-Phe-Trp-NH₂
- Pop-Tyr-Tyr-Aa-Lys-Val-Phe-Trp-NH₂, et
- Aa-Cys(Acm)-Tyr-D-Trp-Lys-Val-Cys(Acm)-Trp-NH₂.

6. Procédé pour la préparation des composés selon les revendications 1 à 5, caractérisé en ce que, lors de l'utilisation de la méthode de synthèse des polypeptides en phase solide, les aminoacides protégés sont graduellement condensés sur le véhicule résine solide par couplage au carbodiimide ou ester actif, dans l'ordre correspondant, puis le produit final est séparé du véhicule solide par clivage acide ou basique, et les groupes protecteurs sont séparés des aminoacides, avant, pendant, ou après le clivage à partir de la résine, et, si désiré, le dérivé octapeptidyl ou heptapeptidyl amide de formule générale I ainsi obtenu est converti en un sel d'addition d'acide, en le faisant réagir avec un acide et/ou si désiré, la base libre est libérée à partir du sel d'addition d'acide, en le faisant réagir avec une base, et, si désiré, cette dernière est convertie en un autre sel d'addition d'acide, en la faisant réagir avec un acide.

7. Procédé selon à revendication 6, caractérisé en ce qu'une résine benzhydrylamine, ou une résine polystyrène chlorométhylée est utilisée comme véhicule résine solide.

8. Procédé selon les revendications 6 ou 7, caractérisé en ce que le produit final contenant les groupes protecteurs est scindé par le gaz fluorhydrique et/ou par ammonolyse.

9. Médicaments caractérisés en ce qu'ils contiennent comme ingrédient(s) actif(s), un ou plusieurs composés selon les revendications 1 à 8, convenablement, en mélange avec un ou plusieurs véhicule(s), et/ou des additifs communément utilisés en technique pharmaceutique.

10. Utilisation des composés selon les revendications 1 à 5, pour la préparation de médicaments, en particulier pour le traitement des mammiféres, y compris l'homme, souffrant de maladie tumorale, et/ou pour l'inhibition de leur activité tyrosine kinase.

## Revendications (Revendications pour l'(les) Etat(s) contractant(s) suivant(s): ES, GR)

1. Procédé pour la préparation de dérivés octapeptidyl ou heptapeptidyl amide de formule générale : 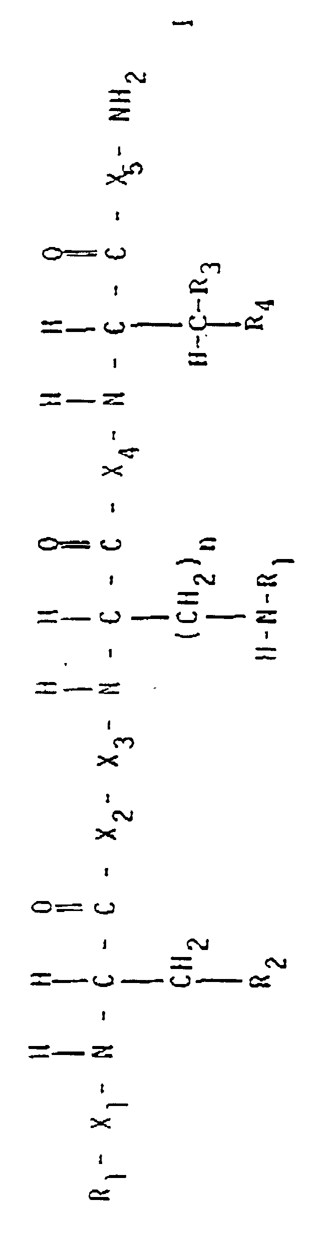 dans laquelle :
- X₁ représente un reste D-aminoacide aromatique, ou un derive de celui-ci substitué sur le cycle par un halogène et/ou un groupe hydroxyle; ou un groupe D-phénylglycyle, p-hydroxyphénylglycyle, o-aminobenzoyle, m-aminobenzoyle, D-tétrahydroisoquinoléylcarbonyle, ou sarcosylanalyle; ou un groupe : 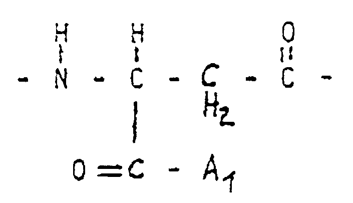 dans lequel :
A₁ représente le reste d'une amine aromatique, hétérocyclique, ou aliphatique comportant un groupe amine primaire ou secondaire, ou
représente un groupe amine aromatique, hétérocyclique, ou aliphatique, primaire ou secondaire,
ou
représente un groupe alcoxy, aryloxy, ou aralkyloxy;
- X₂ représente un reste aminoacide aromatique, ou un dérivé de celui-ci, substitué sur le cycle par un halogène et/ou un groupe hydroxyle; ou un groupe histidyle;
- X₃ représente un groupe D-tryptophyle, o-aminobenzoyle, m-aminobenzoyle, aspartyle, D-aspartyle, β-aspartyle, β-D-aspartyle, ou aminosuccinyle; ou un groupe : 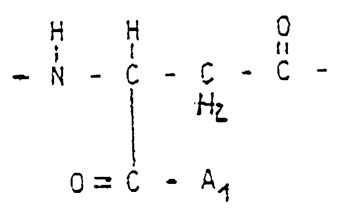 dans lequel :
A₁ est tel que défini ci-dessus;
- X₄ représente un reste aminoacide portant une chaîne latérale alkyle ou aralkyle, ou un dérivé de celui-ci, substitué par un groupe hydroxyle ou méthyle en position β; ou un groupe prolyle ou β-alanyle; ou une liaison de valence;
- X₅ représente un reste aminoacide hétérocyclique, ou un dérivé de celui-ci, substitué sur le cycle par un halogène ou un groupe hydroxyle; ou un groupe thréonyle;
- R₁ représente un atome d'hydrogène ou un groupe : 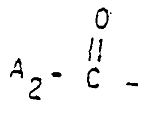 dans lequel :
A₂ représente un atome d'hydrogène ou un groupe alkyle C₁₋₄, éventuellement substitué par un halogène;
- R₂ représente un atome d'hydrogène, un groupe -S-, ou un groupe -S-acétamidométhyle, phényle, ou p-hydroxyphényle;
- R₃ représente un atome d'hydrogène ou un groupe hydroxyle;
- R₄ représente un groupe phényle ou p-hydroxyphényle; ou un groupe -S-, ou -S-acétamidométhyle lorsque R₂ représente le même groupe; ou un groupe méthyle lorsque R₃ est un groupe hydroxyle, dans le cas où R₂ et R₄ sont chacun un groupe -S-, et une liaison de l'un est unie à une liaison de l'autre en une liaison unique représentant un pont entre R₂ et R₄, et
- n est égal à 1, 2, 3 ou 4,
à condition que :
a) si
X₁ représente un groupe D-β-Nal ou D-Phe, éventuellement substitué par un halogène ou un groupe hydroxy,
X₂ représente un groupe Tyr ou Phe
X₃ représente un groupe D-Trp
X₅ représente un groupe Trp ou Thr, et
R₂ et R₄ représentent chacun un groupe -S-, une liaison de l'un étant unie à une liaison de l'autre en une liaison unique représentant un pont entre R₂ et R₄
X₄ est un groupe différent de Val, Thr, ou Ser,
b) si
X₄ représente un groupe Val, ou Thr, ou Ser, et
X₁ représente un résidu D-aminoacide aromatique, ou un dérivé de celui-ci, substitué sur le cycle par un halogène ou un groupe hydroxy,
X₂ représente un reste aminoacide aromatique, ou un dérivé de celui-ci substitue sur le cycle par un halogene ou un groupe hydroxy,
au moins une des conditions suivantes soit remplie :
α) X₃ est un groupe différent de D-Trp, ou
β) R₂ et R₄ ensemble, représentent un groupe différent d'un groupe -S-S- (pont disulfure),
et les sels d'addition d'acide de ceux-ci,
caractérisé en ce que, lors de l'utilisation de la méthode de synthèse des polypeptides en phase solide, les aminoacides protégés sont graduellement condensés sur le véhicule résine solide par couplage au carbodiimide ou ester actif dans l'ordre correspondant, puis le produit final est séparé du véhicule solide par clivage acide ou basique, et les groupes protecteurs sont séparés des aminoacides, avant, pendant, ou après le clivage à partir de la résine, et, si désiré, le dérivé octapeptidyl ou heptapeptidyl amide de formule générale I ainsi obtenu est converti en un sel d'addition d'acide, en le faisant réagir avec un acide et/ou si désiré, la base libre est libérée à partir du sel d'addition d'acide, en le faisant réagir avec une base, et, si désiré, cette dernière est convertie en un autre sel d'addition d'acide, en la faisant réagir avec un acide.

2. Procédé selon la revendication 1, caractérisé en ce qu'une résine benzhydrylamine, ou une résine polystyrène chlorométhylée est utilisée comme véhicule résine solide.

3. Procédé selon les revendications 1 ou 2, caractérisé en ce que le produit final contenant les groupes protecteurs est scindé par le gaz fluorhydrique et/ou par ammonolyse.

4. Procédé selon les revendications 1 à 3, pour la préparation de dérivés octapeptidyl ou heptapeptidyl amide, caractérisé en ce que le reste D-aminoacide pouvant être représenté par X₁, est un reste D-phénylalanyle, D-naphtylalanyle, ou D-tryptophyle, et/ou le reste aminoacide pouvant être représenté par X₂ est un reste tyrosyle, et/ou l'halogène par lequel le reste du dérivé D-aminoacide pouvant être représenté par X₁, et/ou le reste du dérivé aminoacide pouvant être représenté par X₂, peuvent être substitués sur le cycle, est l'iode, par préférence particulière, le reste du dérivé D-aminoacide pouvant être représenté par X₁, étant un reste D-diiodotyrosyle, et/ou le reste de dérivé aminoacide pouvant peut être représenté par X₂, étant un reste diiodotyrosyle.

5. Procedé selon les revendications 1 à 3, pour la préparation de dérivés octapeptidyl ou heptapeptidyl amide, caractérisé en ce que le reste de l'amine pouvant être représenté par A₁, est un reste indolinyle ou phénylamino, le groupe alcoxy pouvant être représenté par A₁, est tel qu'il comporte 1 à 4 atome(s) de carbone, le groupe aryloxy pouvant être représenté par A₁, est un groupe phényloxy, ou le groupe aralkyloxy pouvant être représenté par A₁, est tel qu'il comporte 1 à 4 atome(s) de carbone dans la partie alkyle, et qu'il a une partie phényle en tant que partie aryle.

6. Procédé selon les revendications 1 à 3, pour la préparation de dérivés octapeptidyl ou heptapeptidyl amide, caractérisé en ce que la chaîne latérale alkyle du reste aminoacide ou de son dérivé, pouvant être représentée par X₄, est telle qu'elle comporte 1 à 4 atome(s) de carbone, ou que la chaîne latérale aralkyle du reste aminoacide ou de son dérivé, pouvant être représentée par X₄, est telle qu'elle comporte 1 à 4 atome(s) de carbone dans la partie alkyle, et qu'elle a une partie phényle en tant que partie aryle, et en particulier, le reste aminoacide pouvant être représenté par X₄ est un reste leucyle, valyle, isoleucyle, norvalyle, norleucyle, alanyle ou séryle, et/ou le reste aminoacide pouvant être représenté par X₅ est un reste tryptophyle ou phénylalanyle, ou l'halogène par lequel le reste du dérivé aminoacide aromatique pouvant être représenté par X₅, peut être substitué, est l'iode, et, par préférence particulière, le reste de dérivé aminoacide aromatique pouvant être représenté par X₅, est un reste tyrosyle ou diiodotyrosyle, et/ou le groupe alkyle C₁₋₄ pouvant être représenté par A₂, est tel qu'il comporte 1 à 2 atomes de carbone, ou l'halogène par lequel le groupe alkyle C₁₋₄, pouvant être représenté par A₂, peut être substitué, est le fluor et/ou n est égal à 3 ou à 4.

7. Procédé selon les revendications 1 à 3, pour la préparation de dérivés octapeptidyl ou heptapeptidyl amide, choisis au sein du groupe comprenant les groupes : 
- β-aspartyl-(indolinyl)-Cys-Tyr-D-Trp-Lys-Val-Cys-Thr-NH2

- D-Phe-Cys-Tyr-D-Trp-Lys-Cys-Thr-NH2
 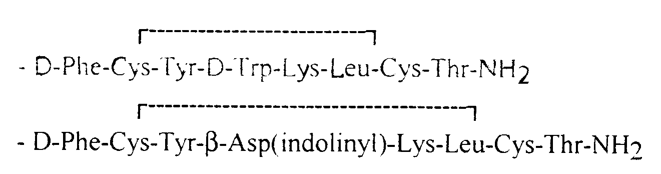
- D-Phe-Cys-Tyr-D-Trp-Lys-Leu-Cys-Thr-NH2
- D-Phe-Cys-Tyr-β-Asp(indolinyl)-Lys-Leu-Cys-Thr-NH2
 - D-Phe-Tyr-Tyr-Aa-Lys-Val-Phe-Trp-NH₂
- Ac-Sar-Ala-Tyr-Tyr-Aa-Lys-Val-Phe-Trp-NH₂
- Pop-Tyr-Tyr-Aa-Lys-Val-Phe-Trp-NH₂, et
- Aa-Cys(Acm)-Tyr-D-Trp-Lys-Val-Cys(Acm)-Trp-NH₂.

8. Procédé de préparation de médicaments caractérisé par le mélange, en tant qu'ingrédient(s) actif(s), d'un ou de plusieurs composés préparés par le procédé selon les revendications 1 à 7, avec un ou plusieurs véhicule(s) et/ou additif(s) communément utilisés en technique pharmaceutique d'une manière connue per se.
